(19) Europäisches Patentamt

European Patent Office

Office européen des brevets

(11) EP 0 758 255 B1

(12) EUROPEAN PATENT SPECIFICATION

(45) Date of publication and mention
of the grant of the patent:
**14.08.2002   Bulletin 2002/33**

(51) Int Cl.$^7$: **A61M 5/315**

(21) Application number: **95917414.5**

(86) International application number:
**PCT/GB95/00982**

(22) Date of filing: **01.05.1995**

(87) International publication number:
**WO 95/30444 (16.11.1995 Gazette 1995/49)**

(54) **LOW DRAG SYRINGE**

REIBUNGSFREIE SPRITZE

SERINGUES A FAIBLE TRAINEE

(84) Designated Contracting States:
**DE DK ES FR GB IE IT SE**

(30) Priority: **06.05.1994  US 239087**
**06.05.1994  US 239092**
**06.05.1994  US 239095**

(43) Date of publication of application:
**19.02.1997   Bulletin 1997/08**

(73) Proprietor: **Amersham Health AS
0401 Oslo (NO)**

(72) Inventors:
• **BASILE, Peter, Anthony
Lawrenceville, NJ 08648 (US)**
• **BROWN, Scott, Cameron
Princeton, NJ 08540 (US)**
• **SNYDER, Fred, Ernest
Princeton Junction, NJ 08550 (US)**
• **TIRRELL, Joseph, Vincent
Birdsboro, PA 19508 (US)**
• **PAGAY, Shrikant, Narayan
Albany, NY 12203 (US)**
• **BACHORIK, Robert, John
B17 Phoenixville, PA 19460 (US)**
• **LIEBERT, Richard, Thomas
Ballston Spa, NY 12020 (US)**

(74) Representative: **Rollins, Anthony John et al
Amersham plc
The Grove Center
White Lion Road
Amersham Buckinghamshire HP7 9LL (GB)**

(56) References cited:
**US-A- 2 895 773         US-A- 3 543 755
US-A- 3 834 387         US-A- 3 939 833**

## Description

This invention relates generally to pre-filled syringes and pre-filled cartridges for administering various fluids into a patient, more particularly, the invention relates to plastic syringes and cartridges for injecting liquid pharmaceutical-biological agents, such as diagnostic-imaging agents, into a patient.

Various syringes for taking body fluid samples or administering fluid medicaments to a patient are known. Such syringes generally include a cylindrical syringe barrel, a hypodermic needle engaged with the syringe barrel, and a plunger within the syringe barrel which, when a force is exerted axially by an operator, create a suction force drawing body fluids into the barrel, or delivers fluid medicament through the hypodermic needle. The purpose of the plunger is to provide an air tight seal between itself and the syringe barrel so that movement of the plunger up and down the barrel will allow liquid, blood or other fluids to be drawn into or forced out of the syringe through the distal end.

Syringes used for such purposes include glass syringes, in which the cylindrical barrel is made of glass and the plunger is a ground glass rod which closely fits within the cylindrical barrel. In order to eliminate leakage and at the same time reduce resistance to an acceptable level, close tolerances are necessary between the barrel and the plunger along with the use of a lubricant. These glass syringes suffer from a number of disadvantages including that: they are expensive since they require close tolerances; they cannot be easily mass produced since the plungers often cannot be interchanged with one another and have to be individually fit with the barrel during the grinding process by the manufacturer; and they are susceptible to breakage.

To obviate these problems syringes were proposed and/or made by using glass and plastic barrels with plastic or elastomeric plungers. In order to prevent leakage around the plunger, the plunger is made with one or more ribs which are slightly larger in diameter in the uncompressed state than the inside of the barrel which upon placement within the barrel are compressed and deformed against the wall of the barrel and thereby form a seal. The quality and strength of the seal depend on the elastomeric properties of the material used to make the plunger and the ratio of the respective diameters of the plunger and the inside of the barrel. To obtain a good leak-proof seal, a relatively large compressive force must be exerted on the elastomeric plunger by the syringe barrel. This quality of seal, however, makes the movement of the plunger within the barrel difficult requiring excessive force on the part of the operator to move the plunger. This drawback is even more pronounced with pre-filled syringes which are maintained, ready to use, in storage. During this shelf-life the plunger tends to seize in the barrel. To remedy the problem the prior art used lubricants to reduce friction and drag between the plunger and the inside of the syringe barrel. One of the commonly used lubricants for this purpose is silicone oil. The use of such lubricants is, however, undesirable, since the lubricants tend to disperse and/or dissolve in parenteral formulations thereby contaminating the formulations. Such potential adulteration is, of course, undesirable and attempts were made to avoid the use of lubricants and still provide a leakage-proof syringe with easily slideable plunger. Such attempts included the use of various plunger configurations including one or more ribs thereon projecting forwardly or rearwardly in the barrel to reduce the frictional drag between the plunger and the barrel. Another approach was, for example in U.S. Patent No. 5,009,646, to laminate the elastomeric plunger with a film of tetrafluoroethylene, ethylenetetrafluoroethylene and ultrahigh molecular weight polyethylene resin.

While liquid tightness and sliding property have somewhat improved with these attempts as regards to syringes intended for taking body fluid samples or injecting medicaments from stored vials, the problem of inadequate sliding property in pre-filled syringes stored for extended time periods still remain unsolved.

It is a main object of at least the preferred embodiments of the present invention to provide a prefilled syringe and a pre-filled cartridge which will overcome the above-described inadequate sliding property while maintaining a tight, leak-proof seal between the plunger and the wall of the syringe barrel.

It is an object of certain preferred embodiments of the present invention to provide a self-aspirating syringe and cartridge.

In medical practice, hypodermic injections are sometimes administered subcutaneously or intramuscularly, while others must be given intravenously, depending upon the particular medication to be administered. In all cases, it is essential that the practitioner knows with certainty, prior to injection of the medication, whether the hypodermic needle tip is located in a major blood vessel, such as a vein, or in subcutaneous tissue. Use of an aspirating syringe in which a negative pressure can be generated in the syringe affords a means of making such determination. Thus the appearance of blood in the syringe upon generation of the negative pressure would indicate location of the needle tip in a major blood vessel, while the lack of appearance of blood would indicate location of the tip in subcutaneous tissue. Depending upon the type of injection intended, the injection can then either proceed directly or if appropriate, the tip can be withdrawn and relocated.

Aspirating syringes are generally of two types, namely, they are either manually or automatically aspirated. In the manually aspirated type the plunger is retracted for a short distance within the barrel of the syringe. This retraction lowers the pressure within the syringe which leaves fluids at the needle tip which are then observable within the barrel of the syringe. From solid tissues no fluids will be drawn into the barrel. In the manually aspirated syringes the injection necessitates the use of both hands, one to hold the barrel, and the other to exert pressure in a rearward direction on the plunger. Such manually actuatable aspirating syringes have the disadvantage that their proper use depends on very large measure on the degree of skill of the person administering the injections.

Aspiration in syringes of the automatic or self-aspirating type is effected by first inducing a positive pressure in a medicament-containing portion of the syringe. On release of the force inducing the positive pressure, a corresponding negative pressure in the syringe is generated thus giving rise to the aspirating effect. Certain preferred embodiments of the present invention relate to the self-aspirating type syringes.

Ideally a self-aspirating hypodermic syringe should be: relatively simple in construction so as to minimize the cost of production; relatively simple to operate; capable of manipulation with one hand; adaptable to multiple self-aspirating actions; and capable of expelling trapped air from the syringe prior to insertion of the needle into the injection site and prior to initiation of the self-aspirating action without either precluding self-aspirating action at a later time in the operation sequence of the syringe or otherwise rendering it inoperative.

The self-aspirating syringes provided by certain preferred embodiments of the present invention mimic, automatically, the slight rearward piston displacement withdrawal action of manually operable syringes, thus generating the slight negative pressure in the syringes essential for aspiration. Such syringes therefore obviate the disadvantage inherent in prior art syringes of the manual type, since the aspirating action is generated automatically which requires no special skill on the part of the practitioner.

The preferred embodiments of the invention will be described in reference to a pre-filled syringe; however, it is to be understood that the invention may be applied to a pre-filled cartridge, having essentially the same shape and other characteristics as a pre-filled syringe. It will therefore be understood that the term "syringe" as used in the claims is intended to cover a cartridge as well as a syringe.

US-A-2 895 773 discloses a syringe comprising a barrel, a plunger and a plunger rod, said plunger rod being actuatable so as to cause axial extension of the plunger. This axial extension causes an increase in the diameter of the plunger, in order to allow the plunger to be used with a barrel of larger diameter.

US-A-3 834 387 discloses a syringe comprising a barrel, a plunger and a plunger rod, said plunger rod being actuatable so as to cause axial extension of the plunger and thereby reduction of the diameter of the plunger. In use, the diameter of the plunger is decreased to create a vent passage around the plunger during insertion thereof into a filled barrel, so that air can escape during the insertion. Once the plunger is in the desired position the deformed plunger is returned to a normal condition in sealing engagement with the barrel.

According to the present invention there is provided a syringe characterised over that of US-A-3 834 387 in that means are provided to limit the axial extension of the plunger such that said reduction in diameter causes a reduction in friction between the inside of the barrel and the plunger, while still maintaining a seal between the inside of the barrel and the plunger.

According to one preferred embodiment, designed to be pre-filled and stored ready for injection,

(a) said barrel has an inner surface defining a cylindrical chamber for retaining an injectable fluid therein; a distal end terminating in a tapered tip to which an injection needle can be attached; and a proximal end for receiving a plunger;

(b) said plunger is cup shaped and slideably mounted in said barrel and positioned close to the proximal end of the barrel to provide a seal with the inner surface of the barrel, said plunger comprising:

(1) a distal convex face which is to interface with the injectable fluid contained in the barrel;
(2) a proximal face;
(3) an outside wall contiguous with the distal convex face having thereon: distal ring, proximal ring and center ring extending radially outwardly and forming a slideable seal with the inner surface of the barrel;
(4) an inside wall;
(5) a bottom rim which together with the inside wall defines a cavity to receive a plunger insert;

(c) said plunger rod has distal and proximal ends, for engaging the plunger and comprises:

(1) a shoulder portion at the distal end of the plunger rod to contact bottom rim of the plunger when plunger rod is inserted in the plunger to limit elongation of the plunger;
(2) a neck portion, contiguous with the plunger rod tip, having male threads thereon to engage female threads of a plunger insert; and
(3) a plunger rod tip, located at the distal end of the plunger rod projecting in the direction of the plunger, the diameter of which is substantially smaller than the diameter of the plunger, and is designed to contact the proximal inside face of the plunger at the center portion thereof; said syringe additionally comprising

(d) a rigid plunger insert having distal end and proximal end positioned within said cavity comprising: an inside wall having female threads thereon to receive and engage male threads on a plunger rod; a flange located at the proximal end having a diameter larger than the diameter of the bottom rim of the plunger so that said plunger insert is securely held within said cavity.

According to a second preferred embodiment, designed to be pre-filled and stored ready for injection,

(a) said barrel has an inner surface defining a cylindrical chamber for retaining an injectable fluid therein; said barrel having distal end terminating in a tapered tip to which an injection needle can be attached; and a proximal end for receiving a plunger;
(b) said plunger is cup-shaped and slideably mounted in said barrel and positioned close to the proximal end of the barrel to provide a seal with the inner surface of the barrel, said plunger comprising:

(1) a distal convex face which is to interface with the injectable fluid contained in the barrel;
(2) a proximal flat or concave face essentially parallel with the distal convex face;
(3) outside wall contiguous with the distal convex face having thereon: distal ring, proximal ring and center ring extending radially outwardly and forming a slideable seal with the inner surface of the barrel;
(4) inside wall having female threads thereon;
(5) bottom rim which together with the inside wall defines a circular opening in the cup-shaped plunger through which a plunger rod can be inserted for engagement; and

(c) said plunger rod has distal and proximal ends, for engaging the plunger and comprises:

(1) a plunger rod tip, located at the distal end of the plunger rod, having a semi-circular shape with convex face projecting in the direction of the plunger, the diameter of which is substantially smaller than the diameter of the plunger, and is designed to contact the proximal flat or concave inside face of the plunger at the center portion thereof;
(2) neck portion, contiguous with the plunger rod tip, designed to receive a slideable cylinder;
(3) slideable cylinder, positioned around the neck portion, comprising: an inside wall and an outside wall, the inside wall defines a cylinder the diameter of which is smaller than the diameter of the plunger rod tip so as to prevent the slideable cylinder slipping off of the neck portion, the outside wall having male threads for engagement of female threads of the plunger when the plunger rod is inserted into the plunger for operation of the syringe.

According to a third preferred embodiment, designed to be pre-filled and stored ready for injection,

(a) said barrel has an inner surface defining a cylindrical chamber for retaining an injectable fluid therein; said barrel having distal end terminating in a tapered tip to which an injection needle can be attached; and a proximal end for receiving a plunger;
(b) said plunger is cup-shaped and slideably mounted in said barrel and positioned close to the proximal end of the barrel to provide a seal with the inner surface of the barrel, said plunger comprising:

(1) a distal convex face which is to interface with the injectable fluid contained in the barrel;
(2) a proximal face;
(3) outside wall contiguous with the distal convex face having thereon: distal ring, proximal ring and center ring extending radially outwardly and forming a slideable seal with the inner surface of the barrel;
(4) inside wall having female threads thereon; and
(5) bottom rim which together with the inside wall defines a circular opening in the cup-shaped plunger through which a plunger actuating cylinder is inserted for engagement; said syringe additionally comprising

(c) a plunger actuating cylinder having a distal end and a proximal end, for engaging the plunger comprising:

(1) male threads at the distal end to engage female threads in the plunger; and
(2) a handle at the proximal end; and

(d) said plunger rod has a distal end and a proximal end, fitted into said plunger actuating cylinder comprising:

(1) a semi-circular shaped tip at the distal end with convex face projecting in the direction of the plunger and the diameter of which is substantially smaller than the diameter of the plunger to press against the proximal face of the plunger when pressure is being exerted on the plunger rod;
(2) a knob at the proximal end located outside the plunger actuating cylinder and serving as first stopping means for the plunger rod to limit protrusion of the plunger rod into the proximal face of the plunger; and
(3) a flange also at the proximal end but spaced from the knob and located within the plunger actuating cylinder and serving as second stopping means for the plunger rod to limit the movement of the plunger rod in the direction toward the proximal end of the plunger actuating cylinder;

said first stopping means and second stopping means are designed to limit the movement of the plunger rod within the plunger actuating cylinder to a predetermined length defined by the distance between the first and second stopping means.

Preferred embodiments of the invention will now be described by way of example only and with reference to the accompanying drawings, in which:

FIG. 1    shows an exploded perspective view of a first embodiment of a syringe containing a plunger and plunger insert, with plunger rod removed, according to the present invention;

FIG. 2    shows a longitudinal cross-section of the syringe and plunger taken along the line 2-2 of FIG. 1;

FIG. 3    shows a cross-section of the plunger rod taken along the line 2-2 of FIG. 1;

FIG. 4    shows an exploded cross-sectional view of the plunger and plunger insert taken along the line 2-2 of FIG. 1;

FIG. 5    shows a cross-sectional view of the plunger and plunger insert taken along the line 2-2 of FIG. 1;

FIG. 6    shows a fragmentary cross-sectional view of the syringe, plunger, plunger insert and plunger rod taken along the line 2-2 of FIG. 1;

FIG. 7    shows a perspective view of an assembled syringe according to a second embodiment, containing a plunger and plunger rod, according to the present invention;

FIG. 8    shows a longitudinal fragmentary cross-section of the syringe, plunger, and plunger rod equipped with a slideable cylinder taken along the line 8-8 of FIG. 7 with plunger and plunger rod inserted in the syringe;

FIG. 9    shows an enlarged fragmentary cross-section of the plunger rod of FIG. 7;

FIG. 10    shows an enlarged fragmentary cross-section of the plunger rod and plunger of FIG. 7 without plunger rod interfacing plunger;

FIG. 11    shows an enlarged fragmentary cross-section of the plunger rod and plunger of FIG. 7 in a dynamic representation of the circumferential reduction produced when interfacing takes place between the plunger rod and plunger upon exerting force on the plunger when commencing an injection;

FIG. 12    shows an enlarged fragmentary perspective view of the plunger rod and slidable cylinder of FIG. 7 when plunger rod is partially inserted in slidable cylinder;

FIG. 13    shows an enlarged perspective view of the plunger rod and slidable cylinder of FIG. 7 when plunger rod is completely inserted in slidable cylinder;

FIG. 14    shows a side elevational view of the plunger and slidable cylinder of FIG. 12;

FIG. 15    shows a side elevational view of the plunger and plunger rod of FIG. 13;

FIG. 16    shows a side elevational view of the plunger rod with the slideable cylinder removed;

FIG. 17    shows a side elevational view of the slideable cylinder having male thread means;

FIG. 18    shows a top plan view of the slideable cylinder of FIG. 17;

FIG. 19    shows a longitudinal fragmentary cross section of a typical prior art syringe, plunger and plunger rod;

FIG. 20    shows an enlarged fragmentary cross section of the prior art plunger and plunger rod of FIG. 19;

FIG. 21    shows a perspective view of an assembled syringe according to a third embodiment comprising a syringe barrel, a plunger, a plunger actuating cylinder and a plunger rod according to the present invention;

FIG. 22    shows a longitudinal cross-section of the plunger taken along the line 22-22 of FIG. 21;

FIG. 23    shows a longitudinal cross-section of the plunger actuating cylinder taken along the line 22-22 of FIG. 21;

FIG. 24    shows a longitudinal cross-section of the plunger rod taken along the line 22-22 of FIG. 21;

FIG. 25    shows an enlarged fragmentary cross-section of the plunger actuating cylinder;

FIG. 26    shows a longitudinal cross-section of the assembled syringe comprising the syringe barrel, the plunger, the plunger actuating cylinder and the plunger rod taken along the line 22-22 of FIG. 21 when the plunger rod is in a base position;

FIG. 27    shows a longitudinal cross-section of the assembled syringe comprising the syringe barrel, the plunger, the plunger actuating cylinder and the plunger rod taken along the line 22-22 of FIG. 21 when the plunger rod is in its extended position;

FIG. 28    shows an enlarged fragmentary cross-section of the plunger, the plunger actuating cylinder and the plunger rod when plunger rod is in its base position; and

FIG. 29    shows an enlarged fragmentary cross-section of the plunger, the plunger actuating cylinder and the plunger rod when the plunger rod is in its extended position.

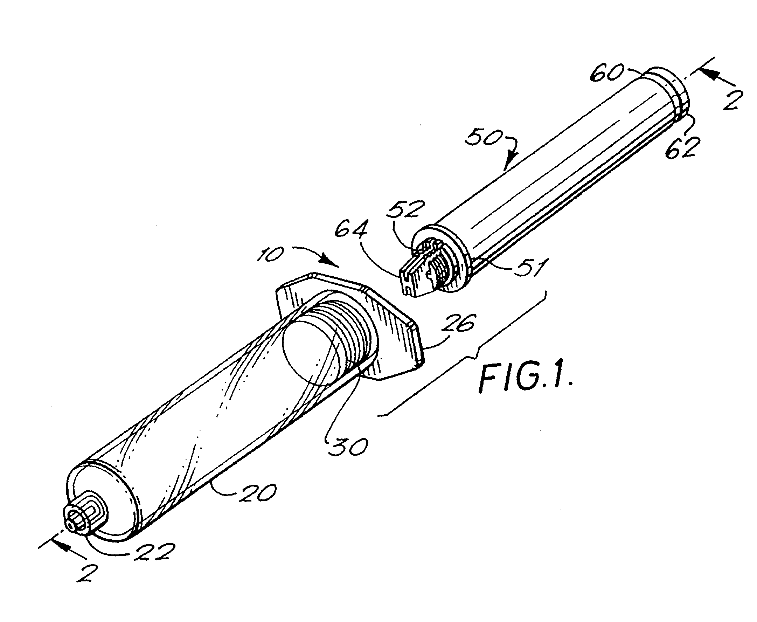

Referring to FIG. 1, there is shown a syringe according to a first embodiment of the present invention generally designated 10 comprising:

> barrel 20;
> plunger 30; and
> plunger rod 50.

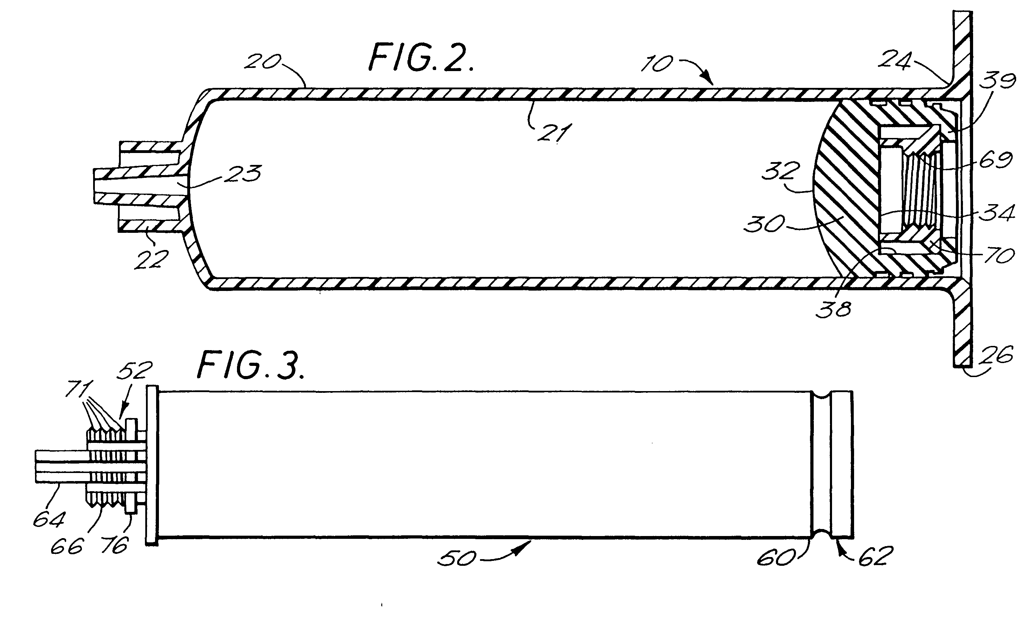

Referring to FIGS. 1,2 and 3, syringe 10 comprises: a barrel 20 having inside wall 21, a distal end terminating in a tapered tip 22 which has bore 23 therethrough, and a proximal end 24 to receive plunger 30; plunger 30 slideably positioned in barrel 20; and plunger rod 50 is attachable to plunger 30. Disposed about the periphery of proximal end 24 of barrel 20 is finger hub 26 which facilitates holding barrel 20 during operation of plunger 30 by exerting a force on plunger rod 50. Plunger rod 50, having distal end 52 and proximal end 60 comprises handle 62 to facilitate exertion of force on plunger 30 by plunger rod 50 during operation of the syringe.

Syringe barrel 20 is made of an inert gas impermeable material, such as glass. However, it is preferably made of a substantially transparent material that is somewhat more flexible than glass, such as polyethylene, polypropylene, polystyrenes, acrylic and methacrylic polymers.

Plunger 30 is made of a compressible, elastomeric material, such as polyisoprene rubber. Plunger rod 50 is made essentially of the same material as the barrel.

Referring to FIGS. 1, 2, 3, 4 and 6, plunger 30 is slideably received in barrel 20 and is moved axially in the barrel by a manual force exerted on plunger rod 50 which is engageable with plunger 30. Plunger 30 in its relaxed state resembles an inverted cup having: a distal convex outside face 32; a proximal inside face 34; outside wall 36 contiguous with distal convex outside face 32; inside wall 38 contiguous with inside face 34; and bottom rim 39 which defines the circular opening in the cup shaped plunger 30. Distal convex face 32 of plunger 30 is to interface with a fluid contained in barrel 20.

Outside wall 36 of plunger 30 comprises: distal ring 40, proximal ring 41, and center ring 42, which are elastically deformable and extend radially outwardly from outside wall 36 and have, when taken together with plunger 30, a minimal diameter slightly in excess of the largest diameter of the working section of barrel 20. The rings form a sealing but slideable engagement with inside wall 21 of barrel 20.

Inside wall 38, inside face 34, and bottom rim 39 define a three-dimensional cavity or area to receive plunger insert 68 of plunger 30.

Plunger insert 68 made of a rigid polymeric material, such as polyethylene, polypropylene, polystyrenes, acrylic and methacrylic polymers comprises: outside wall 74, inside wall 72 having thereon female thread 69 to receive, and engage with, male threads 71 of plunger rod 50; and flange 70. Plunger insert 68 is located in plunger 36 in such a way that the three-dimensional area defined by inside wall 38, inside face 34, and bottom rim 39 completely embraces plunger insert 68. However, the size of plunger insert 68 is such that it does not exert pressure on inside wall 38 so that outside wall of plunger 36 has sufficient space to extend in the lateral direction away from the inside wall of the syringe barrel. Flange 70 of plunger insert 68 has a diameter that is larger than the diameter of bottom rim 39 of plunger 30 so that plunger insert 68 cannot slip out from plunger 30.

Plunger rod 50 comprises: handle 62 located at proximal end 60 thereof to facilitate exertion of manual force thereon by an operator; distal end 52 having male threads 71 thereon designed to engage female threads 69 of plunger insert 68; shoulder 51; plunger rod tip 64 projecting in the direction of the plunger and extending axially forwardly from the threaded portion to form a protrusion. The diameter of said plunger rod tip is substantially smaller than the diameter of the plunger and is designed to contact the proximal inside face 34 at the center portion of plunger 30 upon exertion of pressure on plunger rod 50.

The operation of this embodiment is as follows.

Plunger 30 is inserted into barrel 20 at the proximal end 24 thereof past finger hub 26 so that barrel 20 may be placed pointing vertically upward with its proximal end on a flat surface, such as a filling line, without interference from plunger 30. Barrel 20 is filled with the desired liquid, such as a medicament or a diagnostic imaging medium, through bore 23 in tapered tip 22 and capped. The liquid could be pre-sterilized in bulk and filled into the syringe barrel using aseptic technique or the pre-filled syringe may be sterilized by autoclaving or other means at this point.

The pre-filled, sterilized syringe is then packaged separately from a hypodermic needle and plunger rod to be assembled just prior to use. Preparatory for injection, a hypodermic needle is snapped onto the tapered distal end of the barrel. Plunger rod 50 is threaded into plunger insert 68 contained in the inside cavity of plunger 30 and tightened. The practitioner then gains entry into the desired mammalian site, such as a blood vessel, using conventional venipuncture technique. Female threads 69 of plunger insert 68 engage male threads 71 of plunger rod 50, and plunger rod tip 64 contacts inside face 34 of plunger 30.

This action causes plunger 30 to extend axially: plunger rod 50 forces plunger insert 68 towards the distal end of plunger 30. Plunger insert 68 is limited in its travel because it is pressed against the inside of bottom rim 39 of plunger 30. This axial extension reduces the diameter of the plunger, which reduces the force required to deliver the liquid from the syringe. Further force reduction is achieved when plunger rod tip 64 is forced towards the distal end of the syringe: the force distorts convex face 32 of plunger 30. The distortion, however, is limited when shoulder 51 of plunger rod 50 contacts bottom rim 39 of plunger 30. This contact between shoulder 51 and bottom rim 39 compresses the proximal end of the plunger and thereby limits the elongation of the plunger during activation.

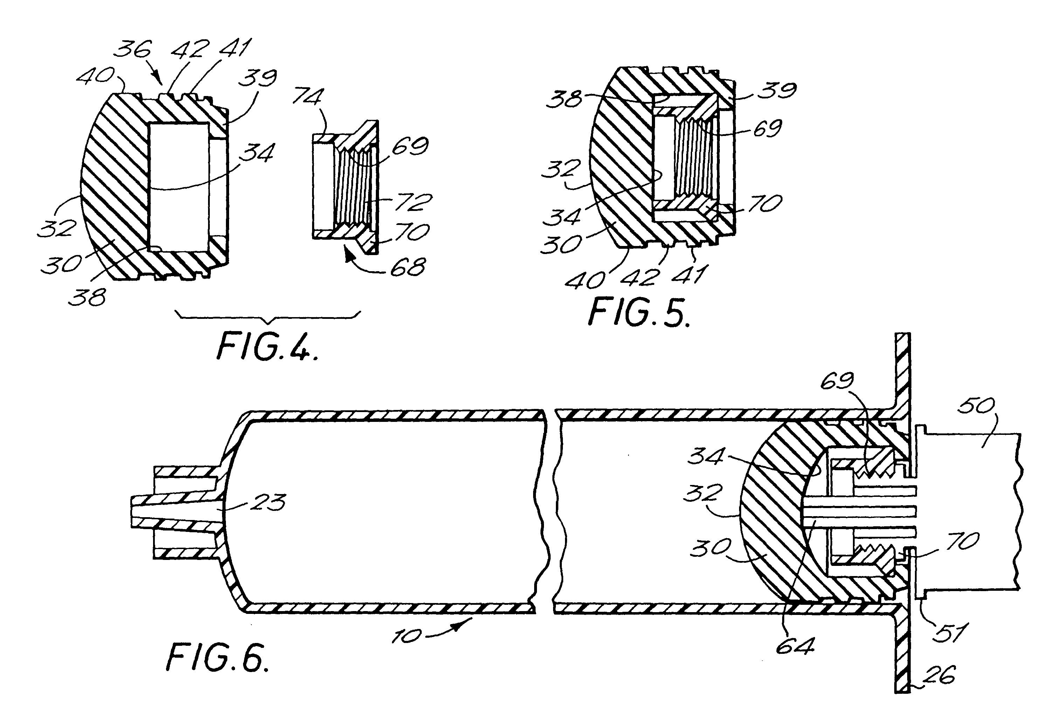

When the operator exerts a relatively slight pressure on plunger rod 50 in the distal direction, the following circumferential deformation of the plunger takes place as shown in FIG. 6: convex face 32 of plunger 30 is extended distally while distal ring 40, proximal ring 41 and center ring 42 are pulled inward by elastic tension forces. This circumferential deformation of the plunger expels head gas from the syringe, i.e. aspirates the syringe. Upon releasing the pressure applied on plunger rod 50, plunger 30 returns to its static position thereby creating a vacuum in barrel 20 and drawing body fluid from the patient indicating that the desired site had been entered and the injection may commence. Aspiration may also be accomplished by pulling the plunger via the plunger rod toward the proximal end of the barrel. By pulling on the plunger rod, plunger insert 68 exerts force only against bottom rim 39 of plunger 30 and bottom rim 39 becomes the leading edge of plunger 30 during this kind of aspiration. As such, the plunger is axially elongated allowing easy manual aspiration.

Upon completing the aspiration process, the operator exerts pressure on plunger rod 50. Referring to proximal ring 41, distal ring 40 and center ring 42, it is clear that the force they now exert on the inside wall 21 of barrel 20 is reduced in direct proportion to the rings' circumferential deformation. As a result, the plunger moves relatively easily in the barrel allowing convenient delivery of the liquid into the injection site. This advantage is even more pronounced when the prefilled syringe is kept in storage for extended time periods during which time the plunger tends to seize in the barrel and the interfacial force between the plunger and the inside wall of the barrel is extremely difficult to break in the axial direction. In the syringe described above the force exerted on the plunger pulls the distal, proximal and center rings inwardly and greatly reduces the interfacial force between the plunger and the inside wall of the barrel.

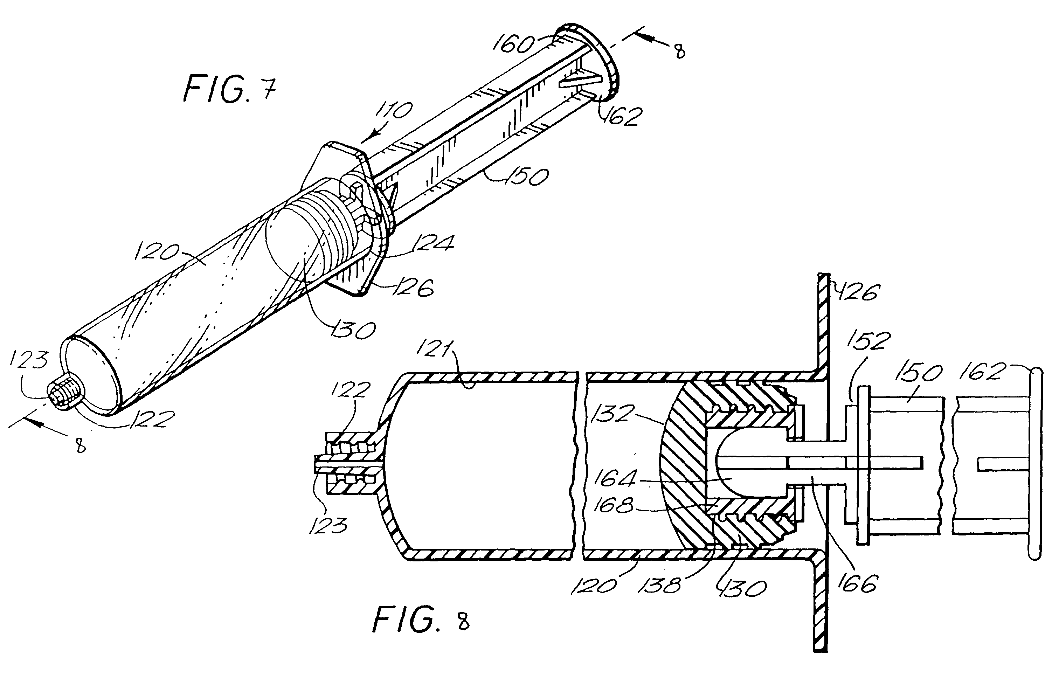

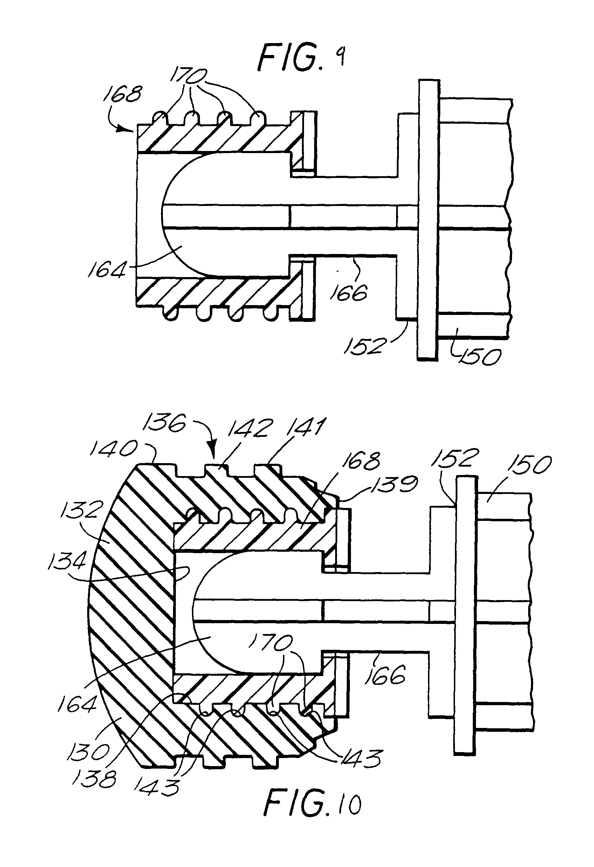

Referring now to FIGS. 7 and 8, there is shown a syringe according to a second embodiment of the present invention, designated 110 comprising: a barrel 120 having inside wall 121, a distal end terminating in a tapered tip 122 which has bore 123 therethrough, and a proximal end 124 to receive plunger 130; plunger 130 slideably positioned in barrel 120; and plunger rod 150 is attachable to plunger 130. Disposed about the periphery of proximal end 124 of the barrel 120 is finger hub 126 which facilitates holding barrel 120 during operation of plunger 130 by exerting a force on plunger rod 150. Plunger rod 150, having distal end 152 and proximal end 160 comprises handle 162 to facilitate exertion of force on plunger 130 by plunger rod 150 during operation of the syringe.

Syringe barrel 120 is made of an inert gas impermeable material such as glass. However, it is preferably made of a substantially transparent material that is somewhat more flexible than glass, such as polyethylene, polypropylene, polystyrenes, acrylic and methacrylic polymers.

Plunger 130 is made of a compressible, elastomeric material, such as polyisoprene rubber. Plunger rod 150 is made essentially of the same material as the barrel.

Referring to FIGS. 7, 9 and 10, plunger 130 is slideably received in barrel 120 and is moved axially in the barrel by a manual force exerted on plunger rod 150 which is engageable with plunger 130. Plunger 130 in its relaxed state resembles an inverted cup having: a distal outside convex face 132; a proximal flat or concave inside face 134 essentially parallel with distal convex face 132; outside wall 136 contiguous with distal convex outside face 132; inside wall 138 contiguous with said flat or concave inside face 134; and bottom rim 139 which defines the circular opening in the cup shaped plunger 130. Distal convex face 132 of plunger 130 is to interface with a fluid contained in barrel 120.

Outside wall 136 of plunger 130 comprises: distal ring 140, proximal ring 141, and center ring 142, which are elastically deformable and extend radially outwardly from outside wall 136 and have, when taken together with plunger 130, a minimal diameter slightly in excess of the largest diameter of the working section of barrel 120. The rings form a sealing but slideable engagement with inside wall 121 of barrel 120.

Inside wall 138 of plunger 130 comprises female threads 143 to receive male threads 170 of plunger rod 150.

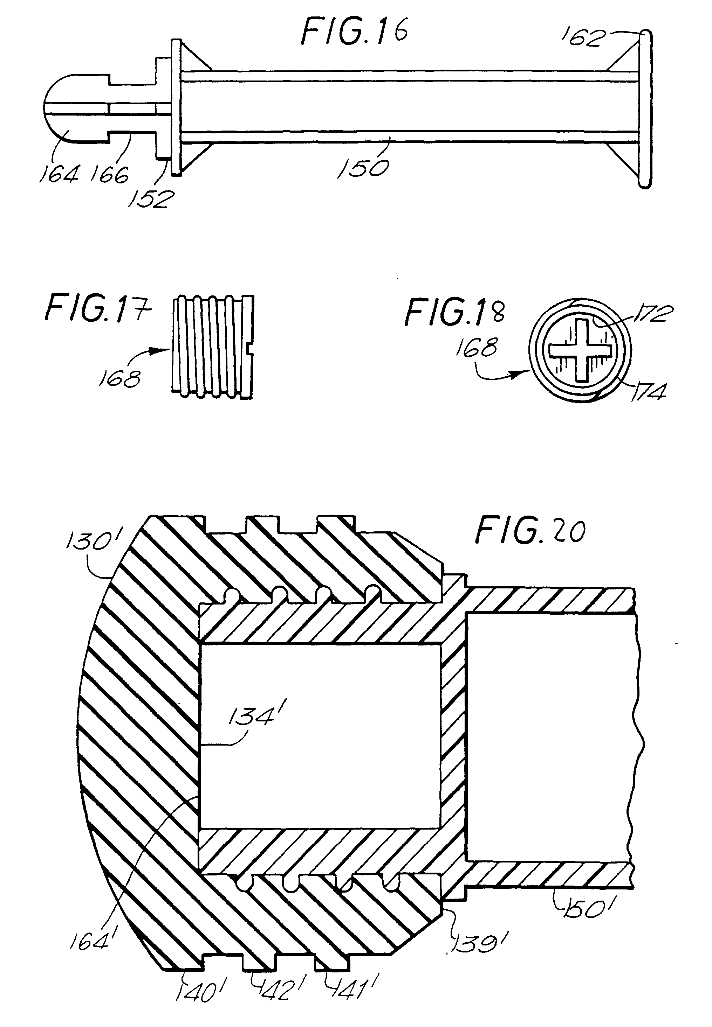

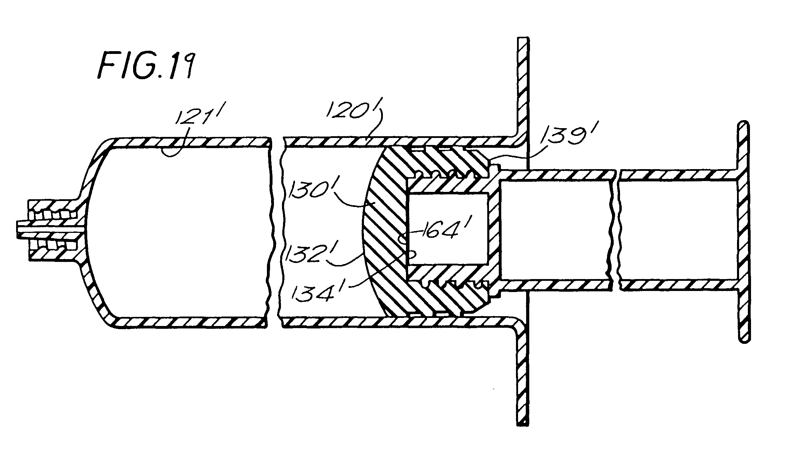

Referring to FIGS. 9, 10, 12, 13, 14, 15, 17 and 18, plunger rod 150 comprises: handle 162 located at proximal end 160 thereof to facilitate exertion of manual force thereon by an operator; plunger rod tip 164 having a semi-circular shape with convex face projecting in the direction of plunger and the diameter of which is substantially smaller than the diameter of the plunger, located at the distal end 152 of plunger rod 150, extending axially forwardly from distal end 152 of plunger rod 150 and is adapted to contact the proximal flat or concave inside face 134 at the center portion of plunger 130 upon exertion of pressure on plunger 130; neck portion 166 of plunger rod 150 located between plunger rod tip 164 and distal end 152 of plunger rod 150 is adapted to receive slideable cylinder 168 which comprises outside wall 174 and inside wall 172. Outside wall 174 has male threads 170 to engage female threads 143 of plunger 130. Inside wall 172 defines a cylinder the diameter of which is somewhat smaller than the diameter of plunger rod tip 164 so as prevent slideable cylinder 168 slipping off the neck portion 166 of plunger rod 150.

The operation of this embodiment is as follows.

Plunger 130 is inserted into barrel 120 of syringe 110 at the proximal end 124 thereof past finger hub 126 so that barrel 120 may be placed pointing vertically upward with its proximal end on a flat surface, such as a filling line, without interference from plunger 130. Barrel 120 is filled with the desired liquid, such as a medicament or a diagnostic imaging medium, by way of bore 123 through tapered tip 122 and capped. The liquid could be pre-sterilized in bulk and filled into the syringe barrel using aseptic technique or the pre-filled syringe may be sterilized by autoclaving or other means at this point.

An alternate filling procedure is to cap the tapered tip 122 and fill the medication from the proximal end of barrel 120. Plunger 130 is then inserted into barrel 120 after filling syringe 110.

The pre-filled, sterilized syringe is then packaged separately from a hypodermic needle to be assembled just prior to use. Preparatory for injection, a hypodermic needle is fitted onto the tapered distal end 122 of barrel 120. Slideable cylinder 168 at the distal end of plunger rod 150 is threaded into plunger 130 and tightened to achieve a snug engagement. The practitioner then gains entry into the desired mammalian site, such as a blood vessel, using conventional venipuncture technique. At this point of the procedure the plunger rod 150 and plunger 130 are in a static engagement. As shown in FIG. 10, female threads 143 of plunger 130 engage male threads 170 of slideable cylinder 168, but plunger tip 164 does not contact flat or concave face 134 of plunger 130 and does not exert pressure thereon.

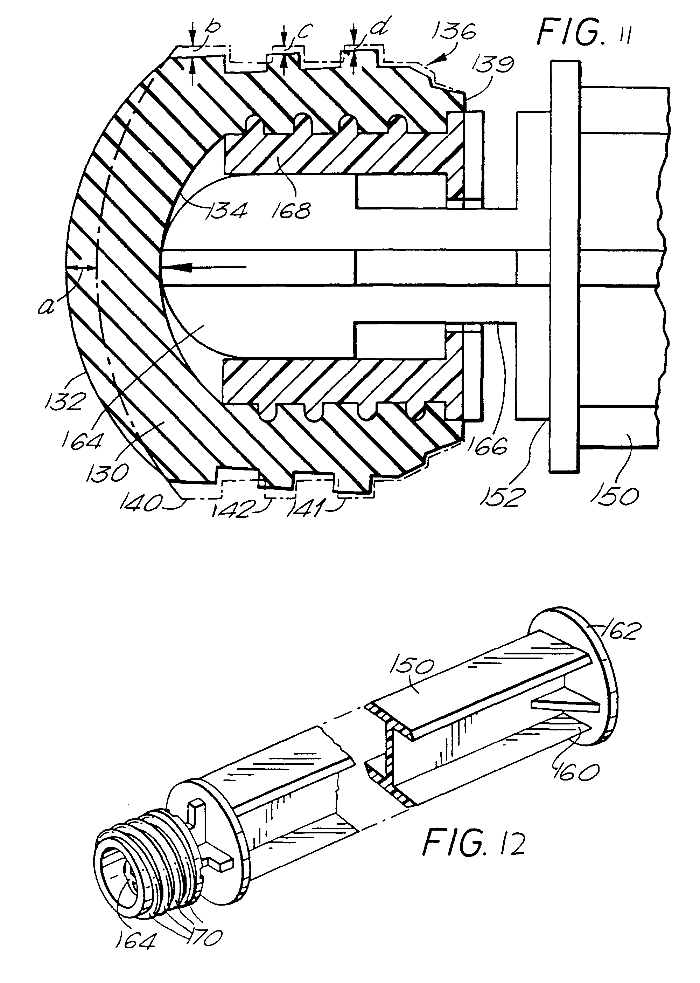

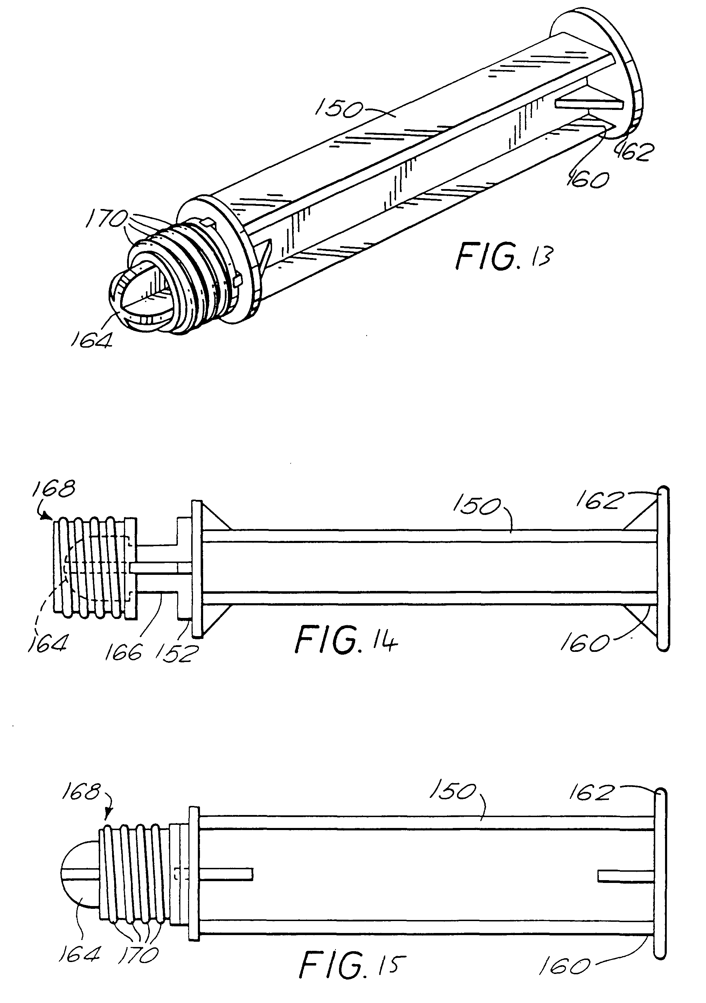

When the operator exerts a relatively slight pressure on plunger rod 150 in the distal direction, the following circumferential deformation of the plunger takes place as shown in FIG. 11: convex face 132 of plunger 130 is extended distally by distance "a" while distal ring 140, proximal ring 141 and center ring 142 are pulled inward by elastic tension forces by distances "b", "c" and "d" respectively. As illustrated, distance "a" is the largest, followed by distances "b", "c" and "d". This circumferential deformation of the plunger expels head gas from the syringe, i.e. aspirates the syringe. Upon releasing the pressure applied on plunger rod 150, plunger 130 returns to its static position thereby creating a vacuum in barrel 120 and drawing body fluid from the patient indicating that the desired site had been entered and the injection may commence. The operator then, again, exerts pressure on plunger rod 150 which results in the same circumferential deformation of plunger 130 as described with respect to aspirating the syringe. Referring to proximal ring 141, distal ring 140 and center ring 142, it is clear that the force they now exert on the inside wall 121 of barrel 120 is reduced in proportion to the distance "b", "c" ind "d" created by the circumferential deformation. As a result, the plunger moves relatively easily in the barrel allowing convenient delivery of the liquid into the injection site. This advantage is even more pronounced when the pre-filled syringe is kept in storage for extended time periods during which time the plunger tends to seize in the barrel and the interfacial force between the plunger and the inside wall of the barrel is extremely difficult to break in the axial direction. In this embodiment of the present invention the force exerted on the plunger pulls the distal, proximal and center rings inwardly and greatly reduces the interfacial force between the plunger and the inside wall of the barrel.

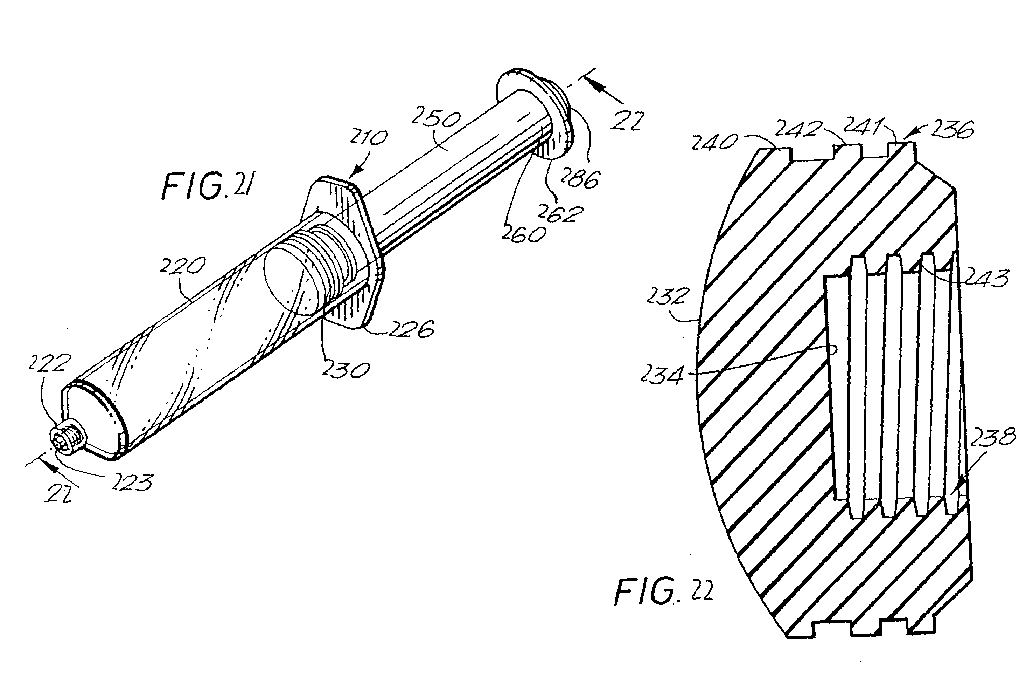

Prior art syringes having generally similar constructions to the present invention are illustrated in FIGS. 19 and 20 wherein corresponding parts are designated with the same numerals with primes (') thereon.

While convex outside face 132' is present, there is no concave inside face 134' in plunger 130', i. e. the inside face 134' of plunger 130' is flat. Plunger rod tip 164' does not exist, but instead, the plunger rod face, which communicates with inside non-convex face 134', is flat. In operation of the prior art syringe, the force exerted on plunger 130' by plunger rod 150' through plunger rod tip 164' will not result in a circumferential reduction of rings 140', 141' and 142' and, consequently, will not result in the reduction of syringing force that is necessary to complete the injection. The illustrated prior art syringe also lacks self-aspirating capability since: upon exertion of force on plunger rod 150', convex face 132' of plunger 130' will not deform, and upon releasing the force, will not regain its static configuration.

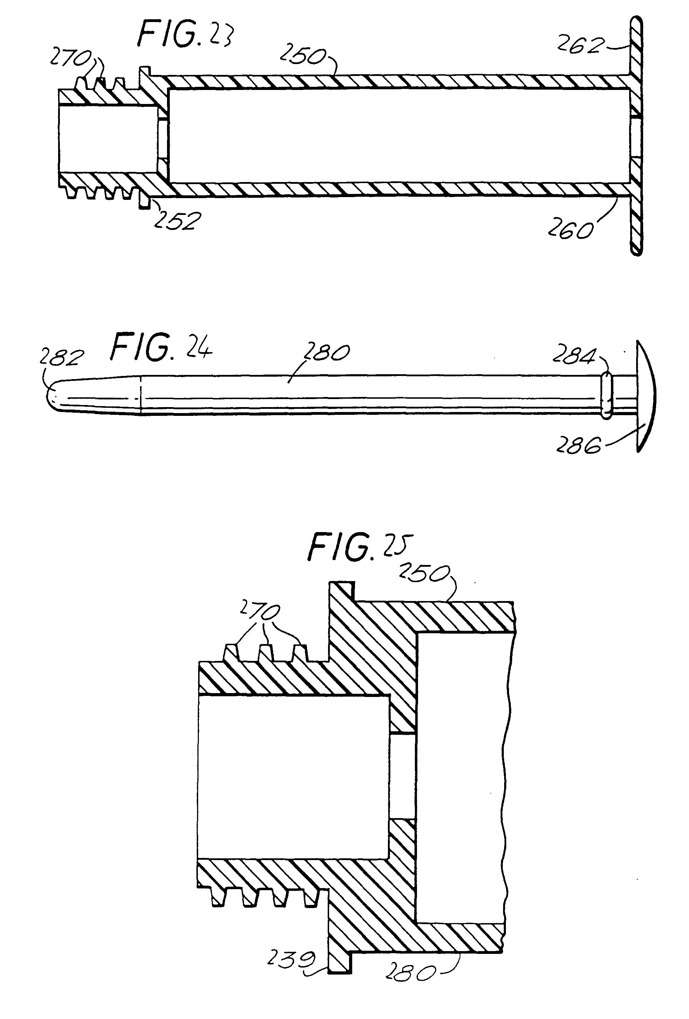

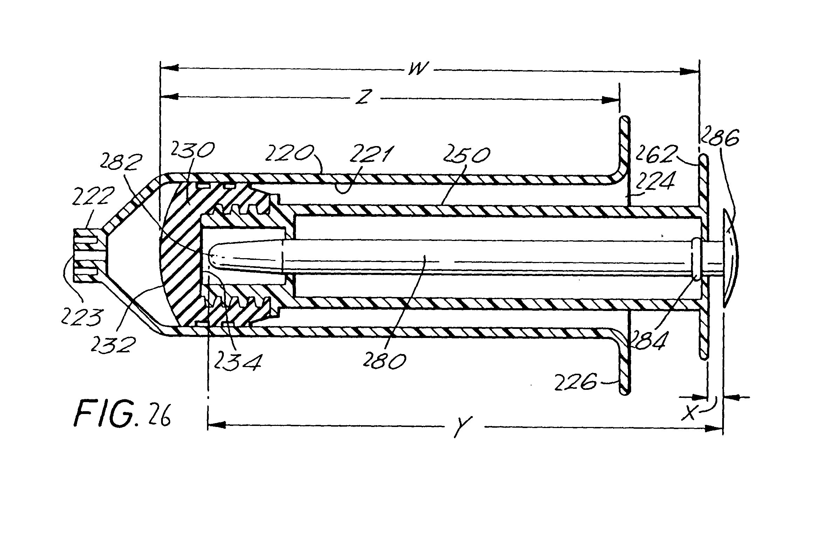

Referring now to FIGS. 21, 22, 23, 24 and 26 there is shown a syringe according to a third embodiment of the present invention, generally designated 210 comprising:

syringe barrel 220 having inside wall 221, distal end terminating in a tapered tip 222 which has a bore 223 therethrough, and proximal end 224 terminating in finger hub 226;

terminating in finger hub 226;

plunger 230 slideably positioned in barrel 220;

plunger actuating cylinder 250 having distal end 252 and proximal end 260, comprising male threads 270 located on the distal end, and handle 262 located on the proximal end thereof, and is designed to engage plunger 230 in barrel 220; and

plunger rod 280 comprises semi-circular plunger rod tip 282 at its distal end, flange 284, and knob 286 at its proximal end, said plunger rod is to be received by said plunger actuating cylinder 250.

Syringe barrel 220 is made of an inert gas impermeable material such as glass. However, it is preferably made of a substantially transparent material that is somewhat more flexible than glass, such as polyethylene, polypropylene, polystyrenes, acrylic and methacrylic polymers.

Plunger 230 is made of a compressible, elastomeric material, such as polyisoprene rubber. Plunger actuating cylinder is made of hard plastic material including polyethylene, polypropylene, polystyrenes, acrylic and methacrylic polymers. Plunger rod 280 is made essentially of the same material as the barrel.

Referring to FIGS. 22, 25, 26 and 27, plunger 230 is slideably positioned in syringe barrel 220 at its proximal end 224 thereof. When plunger 230 is at this position, syringe barrel is placed pointing vertically upward with its distal end on a flat surface, such as filling line, without interference from plunger 230. Syringe barrel 220 is then filled with the desired liquid, such as a medicament or a diagnostic imaging medium through bore 223 in tapered tip 222 and capped. The liquid introduced into the syringe barrel can be pre-sterilized in bulk and filled into the syringe barrel using aseptic technique or the pre-filled syringe may be sterilized by autoclaving or by other means at this point. After the filling process is completed and the syringe is capped, the syringe is packaged to be stored without the syringe actuating cylinder 250 and plunger rod 280 being engaged with the syringe barrel.

An alternate filling procedure is to cap tapered tip 222 and fill the medication from the proximal end of barrel 220. Plunger 230 is then inserted in to barrel 220 after filling syringe 210.

Plunger 230 with sealing means serves to hold the liquid contained in syringe barrel 220 and to variate the interior volume while maintaining a sealing of the interior during aspiration of the syringe and subsequently injection into a patient. As best seen in FIGS. 22 and 25, plunger 230 in its relaxed state resembles an inverted cup having:

a distal outside convex face 232 which is to interface with the liquid contained in syringe barrel 220;

a proximal inside face 234;

outside wall 236 contiguous with distal convex outside face 232;

inside wall 238 contiguous with proximal inside face 234; and

bottom rim 239 which defines a circular opening in the cup-shaped plunger 230.

Outside wall 236 of plunger 230 comprises:

distal ring 240, proximal ring 241 and center ring when taken together with plunger 230, a minimal diameter slightly in excess of the largest diameter of the working section of barrel 220. The rings form a sealing but slideable engagement with inside wall 221 of barrel 220.

Inside wall 238 of plunger 230 comprises: female threads 243 to receive and engage male threads 270 of plunger actuating cylinder 250. Prior to aspiration and injection of liquid into a patient, plunger actuating cylinder 250 is completely threaded into plunger 230 to mesh threads 243 with threads 270.

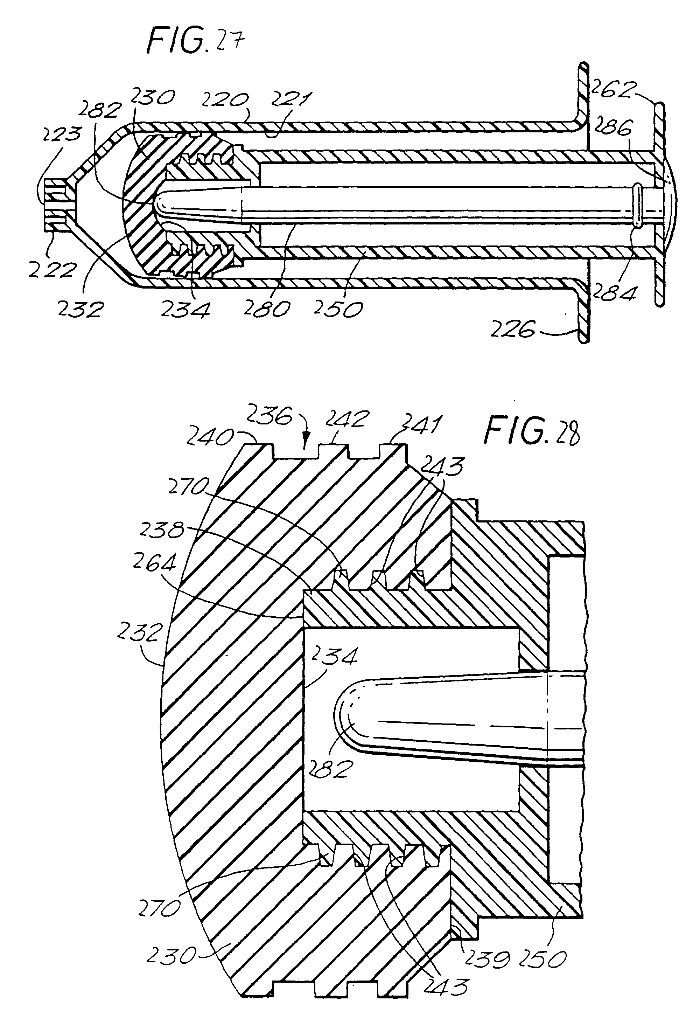

Referring to FIG. 26, plunger rod 280 is inserted into plunger actuating cylinder 250 so that flange 284 is located inside the plunger actuating cylinder, that is, just past handle 262 of the plunger actuating cylinder. Knob 286 and flange 284 of plunger rod 280 serve as stopping means to limit the movement of plunger rod 280 in plunger actuating cylinder 250. Distance X is the distance allowed (which could be called protrusion distance) for movement of the plunger rod in the plunger actuating cylinder controlled by flange 284 and knob 286 which serve as stopping means. The longitudinal cross-section of the assembled syringe shown in FIG. 26 illustrates the various parts of the assembled syringe wherein:

X is the distance between flange 284 and knob 286;

Y is the total length of plunger 280, measured between plunger rod tip 282 and knob 286;

Z is the total length of the functional or working portion of syringe barrel 220; and

W is the total length of plunger actuating cylinder 250 and plunger 230.

W is the total length of plunger actuating cylinder 250 and plunger 230.

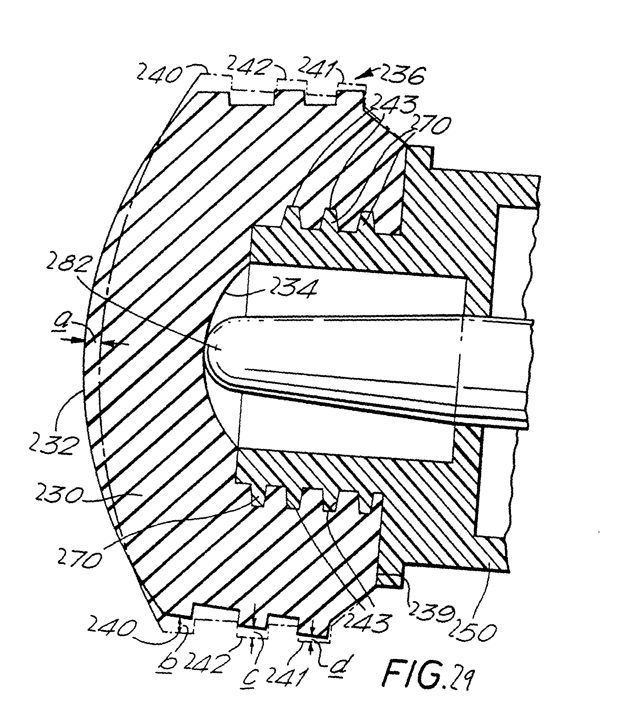

As shown in FIG. 26 and accentuated in FIG 28, the plunger rod is in its base position, that is, plunger rod tip does not reach, and does not exert a force on, the inside face 234 of plunger 230. In this base position flange 284 contacts the handle 262 of plunger actuating cylinder 250.

In FIGS. 27 and 29 the syringe assembly 210 is depicted when plunger rod 280 is in its extended position: knob 286 is pushed against handle 262 and plunger rod tip 282 exerts force on inside face 234 of plunger 230. The force so exerted circumferentially deforms plunger 230: convex outside face 232 of plunger 230 is extended upward for distance "a", while distal ring 240, proximal ring 241 and center ring 242 are pulled inwardly by elastic tension forces for distances "b", "c" and "d" respectively. As illustrated, distance "a" is the largest, followed by distances "b", "c" and "d". This circumferential deformation of the plunger expels head gas from the syringe barrel, i.e. aspirates the syringe. Upon releasing the pressure applied on plunger rod 280, plunger 230 returns to its static position, thereby creating a vacuum in barrel 220 and drawing body fluid from the patient indicating that the desired site had been entered and the injection may commence. The operator then, again, exerts pressure on plunger rod 280 which results in the same circumferential deformation of plunger 230 as described with respect to aspirating the syringe. Referring to proximal ring 241, distal ring 240 and center ring 242, it is clear that the force they now exert on the inside wall 221 of barrel 220 is reduced in proportion to the distances "b", "c" and "d" created by the circumferential deformation. As a result, the plunger moves relatively easily in the barrel allowing periods during which time the plunger tends to seize in the barrel and the interfacial force between the plunger and the inside wall of the barrel is extremely difficult to break in the axial direction. In the embodiment of the present invention the force exerted on the plunger pulls the distal, proximal and center rings inwardly and greatly reduces the interfacial force between the plunger and the inside wall of the barrel.

Experiments were performed to find the most efficient lengths for X, Y, Z and W and their ratios for constructing syringe assemblies typically in use in prefilled syringes. The following illustrates typical ranges: length of:

X:    from 0.010 to 0.750 inches (0.254 to 19.1 mm)
Y:    from 2.000 to 6.000 inches (50.8 to 152.4 mm)
Z:    from 6.000 to 10.000 inches (152.4 to 254 mm) and
W:    from 1.990 to 5.990 inches (50.5 to 152.2 mm)

Their ratios are as follows:

$\frac{X}{Y}$ from 0.375 to 0.002

$\frac{X}{Z}$ from 0.125 to 0.001

$\frac{Y}{Z}$ from 1.000 to 0.200

$\frac{W}{X}$ from 2.650 to 599

$\frac{W}{Y}$ from 0.332 to 2.990

$\frac{W}{Z}$ from 0.199 to 0.998

The protrusion length of plunger rod 280, as defined by the ratio of X to Y, greatly influences the breakaway force of plunger 230 from inside wall 221 of syringe barrel 220.

The operation of this embodiment is as follows.

Plunger actuating cylinder 250, pre-assembled with plunger rod 280, is threaded into plunger 230 contained in the proximal end of syringe barrel 220 so that female threads 243 completely engage male threads 270. Plunger rod 280 at this point is in its base or static position within plunger actuating cylinder 250, that is, flange 284 is positioned against handle 262 of plunger actuating cylinder 250 and plunger rod tip 282 does not exert any pressure on inside face 234 of plunger 230. A hypodermic needle is snapped onto the tip 222 of syringe barrel 220. The practitioner then gains entry into the desired mammalian site, such as a blood vessel, using conventional venipuncture technique. The practitioner then exerts pressure on knob 286 of plunger rod 280 so that knob 286 is pushed against handle 262 of plunger actuating cylinder 250. In turn, tip 284 of plunger rod 280 will be forced against inside face 234 of plunger 230 resulting in a circumferential deformation of the plunger. Convex face 232 of plunger 230 is extended distally for distance "a", while distal ring 240, proximal ring 241 and center ring 242 are pulled inward by elastic tension for distance "b", "c" and "d" respectively. This circumferential deformation of the plunger expels head gas from the syringe, i.e. aspirates the syringe. At the same time the force exerted on the wall 221 of syringe barrel 220 by plunger 230 is greatly reduced facilitating the injection process after aspiration is completed. Upon releasing the pressure applied on plunger rod 280, plunger 230 returns to its original configuration thereby creating a vacuum in syringe barrel 220 and drawing body fluid from the patient indicating that the desired site had been entered and the injection may commence. If body fluid is not drawn, the injection site is to be changed and the aspiration process is to be repeated. At this point the practitioner is ready to inject the fluid into the site, and to that end, exerts pressure on the plunger rod 280 and plunger actuating cylinder 250. The pressure so exerted results in the same circumferential deformation of plunger 230 as described with respect to aspirating the syringe. Referring to proximal ring 241, distal ring 240 and center ring 242, it is clear that the force they now exert on the inside wall 221 of barrel 220 is reduced in direct proportion to the distance "b", "c" and "d" created by the circumferential deformation. As a result, the plunger moves relatively easily in the barrel allowing convenient delivery of the liquid into the injection site. This advantage is even more pronounced when the prefilled syringe is kept in storage for extended time periods during which time the plunger tends to seize in the barrel and the interfacial force between the plunger and the inside wall of the barrel is extremely difficult to break in the axial direction. In this embodiment of the present invention the force exerted on the plunger pulls the distal, proximal and center rings inwardly and greatly reduces the interfacial force between the plunger and the inside wall of the barrel.

It will be appreciated that the syringe of the preferred embodiments of the present invention possesses most of the attributes of an ideal syringe for both aspiration and injection as enumerated above. That is, the syringe is simple in construction, thus minimizing the cost of production; it is simple to operate; it is capable of manipulation with one hand; it is capable of multiple self-aspirating actions with each syringe or cartridge or; and it is capable of expelling air trapped within the syringe or cartridge either prior to initiation of the self-aspirating action or at any time during the sequence of actions necessary for injection of the syringe content without, on the one hand, precluding self-aspirating action at any point in the sequence or, on the other, rendering the self-aspirating action inoperative.

**Claims**

1. A syringe (10,110,210) comprising a barrel (20,120,220), a plunger (30,130,230) and a plunger rod (50,150,280), said plunger rod being actuatable so as to cause axial extension of the plunger and thereby reduction of the diameter of the plunger, **characterised in that** means are provided to limit the axial extension of the plunger such that said reduction in diameter causes a reduction in friction between the inside of the barrel and the plunger, while still maintaining a seal between the inside of the barrel and the plunger.

2. A syringe as claimed in claim 1, wherein the plunger (30,130,230) is substantially cup-shaped with its proximal end (34,134,234) open and its distal end portion (32,132,232) arranged to be axially deformed by actuation of the plunger rod.

3. A syringe as claimed in claim 1 or 2, comprising an insert (68) retained in a cavity of the plunger (30), the insert having a female thread (69), and the plunger rod (50) having a portion at its distal end (52) bearing a male thread (71) adapted to co-operate with the female thread (69) of the insert (68).

4. A syringe as claimed in claim 3, wherein a part (64) of the plunger rod (50) projects beyond the thread-bearing portion (52) and contacts an inner surface (34) of the plunger (30) when the plunger rod (50) is screwed into the insert (68), this projecting part (64) of the plunger rod (50) serving to deform the plunger (30) as the plunger rod is screwed further into the insert.

5. A syringe as claimed in claim 1 or 2, wherein the plunger rod (150) comprises a rod member and a member (168) for attachment to the plunger, the rod member being axially slidable relative to the attachment member (168).

6. A syringe as claimed in claim 5, wherein the attachment member (168) is a tubular member which surrounds the distal end (152) of the rod member.

7. A syringe as claimed in claim 5 or 6, wherein the motion of the rod member is limited by two stops on the distal end of the rod member.

8. A syringe as claimed in any of claims 5 to 7, wherein the rod member is movable relative to the plunger attachment member (168) between a position in which the distal end (164) of the rod member projects beyond the distal end of the plunger attachment member (168), and a position in which the distal end of the rod member (164) does not project beyond the distal end of the plunger attachment member (168).

9. A syringe as claimed in any of claims 5 to 8, wherein the plunger (130) is internally threaded and the plunger attachment member (168) is externally threaded, the threads co-operating to retain the plunger attachment member (168) in the plunger (130).

10. A syringe as claimed in claim 1 or 2, wherein the plunger rod comprises a substantially tubular outer member (250) which surrounds a substantially rod-shaped inner member (280), the inner and outer members (250, 280) being slidable relative to one another.

11. A syringe as claimed in claim 10, wherein the sliding motion of the inner member (280) relative to the outer member (250) is limited by two stops (284,286).

12. A syringe as claimed in claims 10 or 11, wherein the inner member (280) is movable relative to the outer member (250) between a position in which the distal end (282) of the inner member (280) projects beyond the distal end of the outer member (250), and a position in which the distal end (282) of the inner member (280) does not project beyond the distal end of the outer member (250).

13. A syringe as claimed in any of claims 10 to 12, wherein the plunger (230) is internally threaded and the distal end of the outer member (250) is externally threaded, the threads co-operating to retain the outer member in the plunger.

14. A syringe as in any preceding claim, wherein the plunger (30,130,230) has a distal ring (40,140,240), a central ring (42,142,242) and a proximal ring (41,141,241) on its outer circumference, deformation of the plunger serving to draw at least the distal ring (40,140,240) away from the inner surface (21,121,221) of the barrel (20,120,220) while at least the proximal ring (41,141,241) remains in contact with the inner surface of the barrel.

15. A syringe as claimed in claim 1, designed to be pre-filled and stored ready for injection, wherein:

(a) said barrel (20) has an inner surface (21) defining a cylindrical chamber for retaining an injectable fluid therein; a distal end terminating in a tapered tip (22) to which an injection needle can be attached; and a proximal end (24) for receiving a plunger (30);

(b) said plunger (30) is cup shaped and slideably mounted in said barrel and positioned close to the proximal end (24) of the barrel (20) to provide a seal with the inner surface (21) of the barrel (20), said plunger comprising:

(1) a distal convex face (32) which is to interface with the injectable fluid contained in the barrel (20);

(2) a proximal face (34);

(3) an outside wall (36) contiguous with the distal convex face (32) having thereon: distal ring (40), proximal ring (41) and center ring (42) extending radially outwardly and forming a slideable seal with the inner surface (21) of the barrel (20);

(4) an inside wall (38);

(5) a bottom rim (39) which together with the inside wall (38) defines a cavity to receive a plunger insert;

(c) said plunger rod (50) has distal (52) and proximal (60) ends, for engaging the plunger and comprises:

(1) a shoulder portion (51) at the distal end of the plunger rod (50) to contact bottom rim (39) of the plunger (30) when plunger rod (50) is inserted in the plunger (30) to limit elongation of the plunger;

(2) a neck portion (52), contiguous with the plunger rod tip, having male threads (71) thereon to engage female threads of a plunger insert; and

(3) a plunger rod tip (64), located at the distal end of the plunger rod (50) projecting in the direction of the plunger (30), the diameter of which is substantially smaller than the diameter of the plunger (30), and is designed to contact the proximal inside face (34) of the plunger at the center portion thereof; said syringe additionally comprising

(d) a rigid plunger insert (68) having distal end and proximal end positioned within said cavity comprising: an inside wall (72) having female threads (69) thereon to receive and engage male threads on a plunger rod; a flange (70) located at the proximal end having a diameter larger than the diameter of the bottom rim (39) of the plunger (30) so that said plunger insert is securely held within said cavity.

16. A syringe as claimed in claim 1, designed to be pre-filled and stored ready for injection, wherein:

(a) said barrel (120) has an inner surface (121) defining a cylindrical chamber for retaining an injectable fluid therein; said barrel (120) having distal end terminating in a tapered tip (122) to which an injection needle can be attached; and a proximal end (124) for receiving a plunger (130);

(b) said plunger (130) is cup-shaped and slideably mounted in said barrel (120) and positioned close to the proximal end (124) of the barrel (120) to provide a seal with the inner surface (121) of the barrel (120), said plunger comprising:

(1) a distal convex face (132) which is to interface with the injectable fluid contained in the barrel (120);

(2) a proximal flat or concave face (134) essentially parallel with the distal convex face (132);

(3) outside wall (136) contiguous with the distal convex face (132) having thereon: distal ring (140), proximal ring (141) and center ring (142) extending radially outwardly and forming a slideable seal with the inner surface (121) of the barrel (120);

(4) inside wall (138) having female threads (143) thereon;

(5) bottom rim (139) which together with the inside wall (138) defines a circular opening in the cup-shaped plunger through which a plunger rod can be inserted for engagement; and

(c) said plunger rod (150) has distal and proximal ends, for engaging the plunger and comprises:

(1) a plunger rod tip (164), located at the distal end of the plunger rod (150), having a semi-circular shape with convex face projecting in the direction of the plunger, the diameter of which is substantially smaller than the diameter of the plunger, and is designed to contact the proximal flat or concave inside face (134) of the plunger (130) at the center portion thereof;

(2) neck portion (166), contiguous with the plunger rod tip (164), designed to receive a slideable cylinder (168);

(3) slideable cylinder (168), positioned around the neck portion (166), comprising: an inside wall (172) and an outside wall (174), the inside wall (172) defines a cylinder the diameter of which is smaller than the diameter of the plunger rod tip (164) so as to prevent the slideable cylinder (168) slipping off of the neck portion, the outside wall (174) having male threads (170) for engagement of female threads (143) of the plunger (130) when the plunger rod is inserted into the plunger for operation of the syringe.

17. A syringe as claimed in claim 1, designed to be pre-filled and stored ready for injection wherein:

(a) said barrel (220) has an inner surface (221) defining a cylindrical chamber for retaining an injectable fluid therein; said barrel having distal end terminating in a tapered tip (222) to which an injection needle can be attached; and a proximal end (224) for receiving a plunger (230);

(b) said plunger (230) is cup-shaped and slideably mounted in said barrel (220) and positioned close to the proximal end (224) of the barrel (220) to provide a seal with the inner surface (221) of the barrel (220), said plunger comprising:

(1) a distal convex face (232) which is to interface with the injectable fluid contained in the barrel (220);

(2) a proximal face (234);

(3) outside wall (236) contiguous with the distal convex face (232) having thereon: distal ring (240), proximal ring (241) and center ring (242) extending radially outwardly and forming a slideable seal with the inner surface (221) of the barrel (220);

(4) inside wall (238) having female threads thereon; and

(5) bottom rim (239) which together with the inside wall defines a circular opening in the cup-shaped plunger (230) through which a plunger actuating cylinder is inserted for engagement; said syringe additionally comprising

(c) a plunger actuating cylinder (250) having a distal end (252) and a proximal end (260), for engaging the plunger comprising:

(1) male threads (270) at the distal end (252) to engage female threads in the plunger (230); and

(2) a handle (262) at the proximal end; and

(d) said plunger rod (280) has a distal end and a proximal end, fitted into said plunger actuating cylinder comprising:

(1) a semi-circular shaped tip (282) at the distal end with convex face projecting in the direction of the plunger (230) and the diameter of which is substantially smaller than the diameter of the plunger to press against the proximal face (234) of the plunger when pressure is being exerted on the plunger rod (280);

(2) a knob at the proximal end (286) located outside the plunger actuating cylinder (250) and serving as first stopping means for the plunger rod (280) to limit protrusion of the plunger rod into the proximal face of the plunger; and

(3) a flange (284) also at the proximal end but spaced from the knob (280) and located within the plunger actuating cylinder (250) and serving as second stopping means for the plunger rod (280) to limit the movement of the plunger rod in the direction toward the proximal end of the plunger actuating cylinder;

said first stopping means and second stopping means are designed to limit the movement of the plunger rod (280) within the plunger actuating cylinder (250) to a predetermined length defined by the distance between the first and second stopping means.

18. A syringe as claimed any preceding claim, wherein said syringe barrel (20,120,220) is made of an inert gas-impermeable material selected from the group consisting of glass, polyethylene, polypropylene, polystyrenes, acrylic polymers and methacrylic polymers.

19. A syringe as claimed in any preceding claim, wherein said plunger (30,130,230) is made from a compressible, elastomeric material.

**Patentansprüche**

1. Spritze (10, 110, 210), die einen Zylinder (20, 120, 220), einen Kolben (30, 130, 230) sowie eine Kolbenstange (50, 150, 280) umfaßt, wobei die Kolbenstange so betätigbar ist, daß sie eine axiale Ausdehnung des Kolbens und dadurch eine Verringerung des Durchmessers des Kolbens hervorruft, **dadurch gekennzeichnet, daß** Mittel vorgesehen sind, die die axiale Ausdehnung des Kolbens begrenzen, so daß die Verringerung des Durchmessers eine Verringerung der Reibung zwischen der Innenseite des Zylinders und dem Kolben hervorruft, wobei dennoch die Dichtigkeit zwischen der Innenseite des Zylinders und dem Kolben aufrechterhalten wird.

2. Spritze nach Anspruch 1, bei der der Kolben (30, 130, 230) im wesentlichen becherförmig ist, wobei sein proximales Ende (34, 134, 234) offen ist und sein distaler Endabschnitt (32, 132, 232) so beschaffen ist, daß er durch die Betätigung der Kolbenstange axial verformt wird.

3. Spritze nach Anspruch 1 oder 2, die einen in einem Hohlraum des Kolbens (30) gehaltenen Einsatz (68) umfaßt, der ein Innengewinde (69) besitzt, wobei die Kolbenstange (50) an ihrem distalen Ende (52) einen Abschnitt besitzt, der ein Außengewinde (71) trägt, das so beschaffen ist, daß es mit dem Innengewinde (69) des Einsatzes (68) zusammenwirkt.

4. Spritze nach Anspruch 3, bei der ein Teil (64) der Kolbenstange (50) über den das Gewinde tragenden Abschnitt (52) vorsteht und mit einer inneren Oberfläche (34) des Kolbens (30) in Kontakt ist, wenn die Kolbenstange (50) in den Einsatz (68) geschraubt ist, wobei dieser vorstehende Teil (64) der Kolbenstange (50) dazu dient, den Kolben (30) zu verformen, wenn die Kolbenstange weiter in den Einsatz geschraubt wird.

5. Spritze nach Anspruch 1 oder 2, bei der die Kolbenstange (150) ein Stangenelement und ein Element (168) für die Befestigung am Kolben umfaßt, wobei das Stangenelement relativ zu dem Befestigungselement (168) axial gleiten kann.

6. Spritze nach Anspruch 5, bei der das Befestigungselement (168) ein röhrenförmiges Element ist, das das distale Ende (152) des Stangenelements umgibt.

7. Spritze nach Anspruch 5 oder 6, bei der die Bewegung des Stangenelements durch zwei Anschläge am distalen Ende des Stangenelements begrenzt ist.

8. Spritze nach einem der Ansprüche 5 bis 7, bei der das Stangenelement relativ zu dem Kolbenbefestigungselement (168) zwischen einer Position, in der das distale Ende (164) des Stangenelements über das distale Ende des Kolbenbefestigungselements (168) vorsteht, und einer Position, in der das distale Ende des Stangenelements (164) nicht über das distale Ende des Kolbenbefestigungselements (168) vorsteht, beweglich ist.

9. Spritze nach einem der Ansprüche 5 bis 8, bei der der Kolben (130) ein Innengewinde aufweist und das Kolbenbefestigungselement (168) ein Außengewinde aufweist, wobei die Gewinde zusammenwirken, um das Kolbenbefestigungselement (168) im Kolben (130) zu halten.

10. Spritze nach Anspruch 1 oder 2, bei der die Kolbenstange ein im wesentlichen röhrenförmiges äußeres Element (250) umfaßt, das ein im wesentlichen stangenförmiges inneres Element (280) umgibt, wobei die inneren und äußeren Elemente (250, 280) relativ zueinander gleiten können.

11. Spritze nach Anspruch 10, bei der die Gleitbewegung des inneren Elements (280) relativ zum äußeren Element (250) durch zwei Anschläge (284, 286) begrenzt ist.

12. Spritze nach Anspruch 10 oder 11, bei der das innere Element (280) relativ zum äußeren Element (250) zwischen einer Position, in der das distale Ende (282) des inneren Elements (280) über das distale Ende des äußeren Elements (250) vorsteht, und einer Position, in der das distale Ende (282) des inneren Elements (280) nicht über das distale Ende des äußeren Elements (250) vorsteht, beweglich ist.

13. Spritze nach einem der Ansprüche 10 bis 12, bei der der Kolben (230) ein Innengewinde aufweist und das distale Ende des äußeren Elements (250) ein Außengewinde aufweist, wobei die Gewinde zusammenwirken, um das äußere Element im Kolben zu halten.

14. Spritze nach einem vorhergehenden Anspruch, bei der der Kolben (30, 130, 230) auf seinem äußeren Umfang einen distalen Ring (40, 140, 240), einen zentralen Ring (42, 142, 242) und einen proximalen Ring (41, 141, 241) besitzt, wobei die Verformung des Kolbens dazu dient, wenigstens den distalen Ring (40, 140, 240) von der inneren Oberfläche (21, 121, 221) des Zylinders (20, 120, 220) wegzuziehen, während wenigstens der proximale Ring (41, 141, 241) mit der inneren Oberfläche des Zylinders in Kontakt bleibt.

15. Spritze nach Anspruch 1, die so entworfen worden ist, daß sie im voraus befüllt und bereit für die Injektion gelagert werden kann, wobei:

    (a) der Zylinder (20) eine innere Oberfläche (21), die eine zylindrische Kammer definiert, in der ein injizierbares Fluid gehalten wird; ein distales Ende, das in einer konisch zulaufenden Spitze (22) endet, an der eine Injektionsnadel befestigt werden kann; sowie ein proximales Ende (24), das einen Kolben (30) aufnimmt, besitzt;

(b) der Kolben (30) becherförmig ist, in dem Zylinder gleitend angebracht ist und in der Nähe des proximalen Endes (24) des Zylinders (20) positioniert ist, um eine Dichtung mit der inneren Oberfläche (21) des Zylinders (20) zu schaffen, wobei der Kolben umfaßt:

(1) eine distale konvexe Fläche (32), die mit dem in dem Zylinder (20) enthaltenen injizierbaren Fluid eine Grenzfläche bilden soll;

(2) eine proximale Fläche (34);

(3) eine Außenwand (36), die an die distale konvexe Fläche (32) angrenzt und daran aufweist: einen distalen Ring (40), einen proximalen Ring (41) und einen zentralen Ring (42), die sich radial auswärts erstrecken und mit der inneren Oberfläche (21) des Zylinders (20) eine gleitfähige Dichtung bilden;

(4) eine Innenwand (38);

(5) einen Bodenrand (39), der zusammen mit der Innenwand (38) einen Hohlraum definiert, der einen Kolbeneinsatz aufnimmt;

(c) die Kolbenstange (50) für den Eingriff mit dem Kolben ein distales Ende (52) und ein proximales Ende (60) besitzt und umfaßt:

(1) einen Schulterabschnitt (51) am distalen Ende der Kolbenstange (50), der mit dem Bodenrand (39) des Kolbens (30) in Kontakt ist, wenn die Kolbenstange (50) in den Kolben (30) eingesetzt ist, um die Auslenkung des Kolbens zu begrenzen;

(2) einen Halsabschnitt (52), der an die Kolbenstangenspitze angrenzt und daran Außengewinde (71) besitzt, die mit Innengewinden eines Kolbeneinsatzes in Eingriff gelangen; und

(3) eine Kolbenstangenspitze (64), die sich am distalen Ende der Kolbenstange (50) befindet, in Richtung des Kolbens (30) vorsteht, einen Durchmesser besitzt, der wesentlich kleiner als der Durchmesser des Kolbens (30) ist, und so beschaffen ist, daß sie mit der proximalen Innenfläche (34) des Kolbens in seinem Mittelabschnitt in Kontakt ist, wobei die Spritze zusätzlich umfaßt:

(d) einen starren Kolbeneinsatz (68), der ein distales Ende und ein proximales Ende besitzt, im Hohlraum angeordnet ist und umfaßt: eine Innenwand (72) mit Innengewinden (69), um Außengewinde an einer Kolbenstange aufzunehmen und mit diesen in Eingriff zu gelangen; und einen Flansch (70), der sich am proximalen Ende befindet und einen Durchmesser aufweist, der größer als der Durchmesser des Bodenrandes (39) des Kolbens (30) ist, so daß der Kolbeneinsatz im Hohlraum sicher gehalten wird.

16. Spritze nach Anspruch 1, die so beschaffen ist, daß sie im voraus befüllt und bereit für die Injektion gelagert werden kann, wobei:

(a) der Zylinder (120) eine innere Oberfläche (121) besitzt, die eine zylindrische Kammer definiert, in der ein injizierbares Fluid gehalten wird; wobei der Zylinder (120) ein distales Ende, das in einer konisch zulaufenden Spitze (122) endet, an der eine Injektionsnadel befestigt werden kann; sowie ein proximales Ende (124), das einen Kolben (130) aufnimmt, besitzt;

(b) der Kolben (130) becherförmig ist, im Zylinder (120) gleitend angebracht ist und in der Nähe des proximalen Endes (124) des Zylinders (120) positioniert ist, um mit der inneren Oberfläche (121) des Zylinders (120) eine Dichtung zu schaffen, wobei der Kolben umfaßt:

(1) eine distale konvexe Fläche (132), die mit dem in dem Zylinder (120) enthaltenen injizierbaren Fluid eine Grenzfläche bilden soll;

(2) eine proximale ebene oder konkave Fläche (134), die zu der distalen konvexen Fläche (132) im wesentlichen parallel ist;

(3) eine Außenwand (136), die an die distale konvexe Fläche (132) angrenzt und daran ausweist: einen distalen Ring (140), einen proximalen Ring (141) und einen zentralen Ring (142), die sich radial auswärts erstrecken und mit der inneren Oberfläche (121) des Zylinders (120) eine gleitfähige Dichtung bilden;

(4) eine Innenwand (138), an der Innengewinde (143) vorhanden sind;

(5) einen Bodenrand (139), der zusammen mit der Innenwand (138) eine kreisförmige Öffnung in dem becherförmigen Kolben definiert, durch die eine Kolbenstange eingeführt werden kann, um darin in Eingriff zu gelangen; und

(c) die Kolbenstange (150) ein distales Ende und ein proximales Ende besitzt, mit dem Kolben in Eingriff gelangt und umfaßt:

(1) eine Kolbenstangenspitze (164), die sich am distalen Ende der Kolbenstange (150) befindet, halbkreisförmig ist und eine in Richtung des Kolbens vorstehende konvexe Fläche besitzt, deren Durchmesser wesentlich kleiner als der Durchmesser des Kolbens ist, wobei die Kolbenstangenspitze (164) so entworfen ist, daß sie mit der proximalen ebenen oder konkaven Innenfläche (134) des Kolbens (130) in dessen Mittelabschnitt in Kontakt gelangt;

(2) einen Halsabschnitt (166), der an die Kolbenstangenspitze (164) angrenzt und so entworfen ist, daß er einen gleitfähigen Zylinder (168) aufnimmt;

(3) einen gleitfähigen Zylinder (168), der um den Halsabschnitt (166) positioniert ist und umfaßt: eine Innenwand (172) und eine Außenwand (174), wobei die Innenwand (172) einen Zylinder definiert, dessen Durchmesser kleiner als der Durchmesser der Kolbenstangenspitze (164) ist, um so zu verhindern, daß der gleitfähige Zylinder (168) aus dem Halsabschnitt gleitet, wobei die Außenwand (174) Außengewinde (170) besitzt, die mit Innengewinden (143) des Kolbens (130) in Eingriff gelangen, wenn die Kolbenstange in den Kolben eingeschoben ist, um die Spritze zu betätigen.

17. Spritze nach Anspruch 1, die so entworfen ist, daß sie im voraus befüllt und bereit für die Injektion gelagert werden kann, wobei:

(a) der Zylinder (220) eine innere Oberfläche (221) besitzt, die eine zylindrische Kammer definiert, in der ein injizierbares Fluid gehalten wird; wobei der Zylinder ein distales Ende, das in einer konisch zulaufenden Spitze (222) endet, an der eine Injektionsnadel befestigt werden kann; sowie ein proximales Ende (224), das einen Kolben (230) aufnimmt, besitzt;

(b) der Kolben (230) becherförmig ist, in dem Zylinder (220) gleitend angebracht ist und in der Nähe des proximalen Endes (224) des Zylinders (220) positioniert ist, um mit der inneren Oberfläche (221) des Zylinders (220) eine Dichtung zu schaffen, wobei der Kolben umfaßt:

(1) eine distale konvexe Fläche (232), die mit dem im Zylinder (220) enthaltenen injizierbaren Fluid eine Grenzfläche bilden soll;

(2) eine proximale Fläche (234);

(3) eine Außenwand (236), die an die distale konvexe Fläche (232) angrenzt und daran aufweist: einen distalen Ring (240), einen proximalen Ring (241) und einen zentralen Ring (242), die sich radial auswärts erstrecken und mit der inneren Oberfläche (221) des Zylinders (220) eine gleitfähige Dichtung bilden;

(4) eine Innenwand (238), an der Innengewinde vorgesehen sind; und

(5) einen Bodenrand (239), der zusammen mit der Innenwand eine kreisförmige Öffnung im becherförmigen Kolben (230) definiert, durch die ein Kolbenbetätigungszylinder eingeführt wird, um darin in Eingriff zu gelangen; wobei die Spritze zusätzlich umfaßt:

(c) einen Kolbenbetätigungszylinder (250), der ein distales Ende (252) und ein proximales Ende (260) besitzt, mit dem Kolben in Eingriff gelangt und umfaßt:

(1) Außengewinde (270) am distalen Ende (252), die mit Innengewinden im Kolben (230) in Eingriff gelangen; und

(2) einen Griff (262) am proximalen Ende; und

(d) die Kolbenstange (280) ein distales Ende und ein proximales Ende besitzt, in den Kolbenbetätigungszylinder eingepaßt ist und umfaßt:

(1) eine halbkreisförmige Spitze (282) am distalen Ende mit konvexer Fläche, die in Richtung des Kolbens (230) vorsteht und deren Durchmesser wesentlich kleiner als der Durchmesser des Kolbens ist, um gegen die proximale Fläche (234) des Kolbens zu drücken, wenn auf die Kolbenstange (280) ein Druck ausgeübt wird;

(2) einen Knopf am proximalen Ende (286), der sich außerhalb des Kolbenbetätigungszylinders (250) befindet und als erstes Anschlagmittel für die Kolbenstange (280) dient, das den Überstand der Kolbenstange in die proximale Fläche des Kolbens begrenzt; und

(3) einen Flansch (284), der sich ebenfalls am proximalen Ende, jedoch in einem Abstand vom Knopf (280) und innerhalb des Kolbenbetätigungszylinders (250) befindet und als zweites Anschlagmittel für die Kolbenstange (280) dient, um die Bewegung der Kolbenstange in Richtung zum proximalen Ende des Kolbenbetätigungszylinders zu begrenzen;

wobei das erste Anschlagmittel und das zweite Anschlagmittel so entworfen sind, daß sie die Bewegung der Kolbenstange (280) im Kolbenbetätigungszylinder (250) auf eine vorgegebene Länge begrenzen, die durch die Strecke zwischen dem ersten und dem zweiten Anschlagmittel definiert ist.

**18.** Spritze nach einem vorhergehenden Anspruch, bei der der Spritzenzylinder (20, 120, 220) aus einem für Inertgas undurchlässigen Material hergestellt ist, das aus der Gruppe gewählt ist, die aus Glas, Polyethylen, Polypropylen, Polystyrole, Acrylpolymere und Methacrylpolymere besteht.

**19.** Spritze nach einem vorhergehenden Anspruch, bei der der Kolben (30, 130, 230) aus einem komprimierbaren Elastomermaterial hergestellt ist.

**Revendications**

**1.** Seringue (10, 110, 210) comprenant un fût (20, 120, 220), un piston (30, 130, 230) et une tige de piston (50, 150, 280), ladite tige de piston pouvant être actionnée de façon à provoquer une extension axiale du piston, et, par conséquent, une réduction du diamètre du piston, **caractérisée en ce que** des moyens sont présents pour limiter l'extension axiale du piston de telle sorte que ladite réduction de diamètre provoque une réduction de frottement entre l'intérieur du fût et le piston, tout en maintenant toujours une étanchéité entre l'intérieur du fût et le piston.

**2.** Seringue selon la revendication 1, dans laquelle le piston (30, 130, 230) a sensiblement une forme de coupelle avec son extrémité proximale (34, 134, 234) ouverte et sa partie d'extrémité distale (32, 132, 232) configurée de façon à être axialement déformée par l'actionnement de la tige de piston.

**3.** Seringue selon la revendication 1 ou 2, comprenant une pièce d'insertion (68) maintenue dans une cavité du piston (30), la pièce d'insertion comportant un filetage femelle (69), et la tige de piston (50) comportant une partie à son extrémité distale (52) qui porte un filetage mâle (71) adapté pour coopérer avec le filetage femelle (69) de la pièce d'insertion (68).

**4.** Seringue selon la revendication 3, dans laquelle une partie (64) de la tige de piston (50) fait saillie au-delà de la partie portant un filetage (52) et vient en contact avec une surface intérieure (34) du piston (30) lorsque la tige de piston (50) est vissée à l'intérieur de la pièce d'insertion (68), cette partie saillante (64) de la tige de piston (50) servant à déformer le piston (30) lorsque la tige de piston est encore davantage vissée à l'intérieur de la pièce d'insertion.

**5.** Seringue selon la revendication 1 ou 2, dans laquelle la tige de piston (150) comprend un élément de tige et un élément (168) pour la fixation au piston, l'élément de tige pouvant coulisser axialement par rapport à l'élément de fixation (168).

**6.** Seringue selon la revendication 5, dans laquelle l'élément de fixation (168) est un élément tubulaire qui entoure l'extrémité distale (152) de l'élément de tige.

**7.** Seringue selon la revendication 5 ou 6, dans laquelle le déplacement de l'élément de tige est limité par deux butées sur l'extrémité distale de l'élément de tige.

**8.** Seringue selon l'une quelconque des revendications 5 à 7, dans laquelle l'élément de tige peut se déplacer par rapport à l'élément de fixation de piston (168) entre une position dans laquelle l'extrémité distale (164) de l'élément de tige fait saillie au-delà de l'extrémité distale de l'élément de fixation de piston (168), et une position dans laquelle l'extrémité distale de l'élément de tige (164) ne fait pas saillie au-delà de l'extrémité distale de l'élément de fixation de piston (168).

**9.** Seringue selon l'une quelconque des revendications 5 et 8, dans laquelle le piston (130) est fileté intérieurement et l'élément de fixation de piston (168) est fileté extérieurement, les filetages coopérant de façon à maintenir l'élément de fixation de piston (168) dans le piston (130).

**10.** Seringue selon la revendication 1 ou 2, dans laquelle la tige de piston comprend un élément extérieur sensiblement tubulaire (250) qui entoure un élément intérieur sensiblement en forme de tige (280), les éléments intérieur et extérieur (250, 280) pouvant coulisser l'un par rapport à l'autre.

**11.** Seringue selon la revendication 10, dans laquelle le déplacement de coulissement de l'élément intérieur (280) par rapport à l'élément extérieur (250) est limité par deux butées (284, 286).

**12.** Seringue selon les revendications 10 ou 11, dans laquelle l'élément intérieur (280) peut se déplacer par rapport à l'élément extérieur (250) entre une position dans laquelle l'extrémité distale (282) de l'élément intérieur (280) fait saillie au-delà de l'extrémité distale de l'élément extérieur (250), et une position dans laquelle l'extrémité distale (282) de l'élément intérieur (280) ne fait pas saillie au-delà de l'extrémité distale de l'élément extérieur (250).

**13.** Seringue selon l'une quelconque des revendications 10 à 12, dans laquelle le plongeur (230) est fileté intérieurement et l'extrémité distale de l'élément extérieur (250) est filetée extérieurement, les filetages coopérant de façon à maintenir l'élément extérieur dans le piston.

**14.** Seringue selon l'une quelconque des revendications précédentes, dans laquelle le piston (30, 130, 230) comporte une bague distale (40, 140, 240), une bague centrale (42, 142, 242) et une bague proximale (41, 141, 241) sur sa circonférence extérieure, la déformation du piston servant à éloigner au moins la bague distale (40, 140, 240) de la surface intérieure (21, 121, 221) du fût (20, 120, 220) tandis qu'au moins la bague proximale (41, 141, 241) reste en contact avec la surface intérieure du fût.

**15.** Seringue selon la revendication 1, conçue pour être pré-remplie et stockée en étant prête à l'injection, dans laquelle :

(a) ledit fût (20) comporte une surface intérieure (21) définissant une chambre cylindrique pour maintenir un fluide injectable à l'intérieur de celle-ci ; une extrémité distale s'achevant par une pointe effilée (22) à laquelle peut être fixée une aiguille d'injection ; et une extrémité proximale (24) pour recevoir un piston (30) ;

(b) ledit piston (30) est en forme de coupelle et est monté de façon à pouvoir coulisser dans ledit fût, et est positionné à proximité de l'extrémité proximale (24) du fût (20) pour assurer une étanchéité avec la surface intérieure (21) du fût (20), ledit piston comprenant :

(1) une face distale convexe (32) qui sert à venir en interface avec le fluide injectable contenu dans le fût (20) ;
(2) une face proximale (34) ;
(3) une paroi extérieure (36) contiguë à la face distale convexe (32), comportant sur celle-ci : une bague distale (40), une bague proximale (41) et une bague centrale (42) s'étendant radialement vers l'extérieur et constituant un joint coulissant avec la surface intérieure (21) du fût (20) ;
(4) une paroi intérieure (38) ;
(5) un rebord inférieur (39), qui, avec la paroi intérieure (38), définit une cavité pour recevoir une pièce d'insertion de piston ;

(c) ladite tige de piston (50) comporte des extrémités distale (52) et proximale (60) pour s'engager avec le piston, et comprend :

(1) une partie d'épaulement (51) à l'extrémité distale de la tige de piston (50) pour venir en contact avec le rebord inférieur (39) du piston (30) lorsque la tige de piston (50) est insérée dans le piston (30) pour limiter l'allongement du piston ;
(2) une partie de col (52), contiguë à la pointe de tige de piston, comportant des filetages mâles (71) sur celle-ci pour coopérer avec des filetages femelles d'une pièce d'insertion de piston ; et
(3) une pointe de tige de piston (64), disposée à l'extrémité distale de la tige de piston (50), faisant saillie dans la direction du piston (30), dont le diamètre est sensiblement inférieur au diamètre du piston (30), et qui est conçue pour venir en contact avec la face intérieure proximale (34) du piston au niveau de la partie centrale de celui-ci ; ladite seringue comprenant de plus :

d) une pièce d'insertion de piston rigide (68) comportant une extrémité distale et une extrémité proximale positionnées à l'intérieur de ladite cavité, comprenant : une paroi intérieure (72) comportant des filetages femelles (69) sur celle-ci pour recevoir des filetages mâles sur une tige de piston et coopérer avec ceux-ci ; un flasque (70) disposé à l'extrémité proximale, ayant un diamètre supérieur au diamètre du rebord inférieur (39) du piston (30), de telle sorte que ladite pièce d'insertion de piston soit fermement maintenue à l'intérieur de ladite cavité.

**16.** Seringue selon la revendication 1, conçue pour être pré-remplie et stockée en étant prête à l'injection, dans laquelle :

(a) ledit fût (120) comporte une surface intérieure (121) définissant une chambre cylindrique pour maintenir un fluide injectable à l'intérieur de celle-ci ; ledit fût (120) comportant une extrémité distale s'achevant par une pointe effilée (122) sur laquelle peut être fixée une aiguille d'injection ; et une extrémité proximale (124) pour recevoir un piston (130) ;

(b) ledit piston (130) est en forme de coupelle et monté de façon à pouvoir coulisser dans ledit fût (120), et est positionné à proximité de l'extrémité proximale (124) du fût (120) de façon à assurer une étanchéité avec la surface intérieure (121) du fût (120), ledit piston comprenant :

(1) une face distale convexe (132) qui sert à venir en interface avec le fluide injectable contenu dans le fût (120) ;
(2) une face proximale plate ou concave (134) essentiellement parallèle à la face distale convexe (132) ;

(3) une paroi extérieure (136) contiguë à la face distale convexe (132), comportant sur celle-ci : une bague distale (140), une bague proximale (141) et une bague centrale (142) s'étendant radialement vers l'extérieur et formant un joint coulissant avec la surface intérieure (121) du fût (120) ;

(4) une paroi intérieure (138) comportant des filetages femelles (143) sur celle-ci ;

(5) un rebord inférieur (139), qui, avec la paroi intérieure (138), définit une ouverture circulaire dans le piston en forme de coupelle, à travers laquelle une tige de piston peut être insérée pour son engagement ; et

(c) ladite tige de piston (150) comporte des extrémités distale et proximale, pour s'engager avec le piston, et comprend :

(1) une pointe de tige de piston (164), disposée à l'extrémité distale de la tige de piston (150), ayant une forme semi-circulaire avec une face convexe faisant saillie dans la direction du piston, dont le diamètre est sensiblement inférieur au diamètre du piston, et qui est conçue pour venir en contact avec la face intérieure proximale plate concave (134) du piston (130) au niveau de la partie centrale de celui-ci ;

(2) une partie de col (166), contiguë à la pointe de tige de piston (164), conçue pour recevoir un cylindre coulissant (168) ;

(3) un cylindre coulissant (168), positionné autour de la partie de col (166), comprenant : une paroi intérieure (172) et une paroi extérieure (174), la paroi intérieure (172) définissant un cylindre dont le diamètre est inférieur au diamètre de la pointe de tige de piston (164) de façon à empêcher le cylindre coulissant (168) de glisser hors de la partie de col, la paroi extérieure (174) comportant des filetages mâles (170) pour coopérer avec des filetages femelles (143) du piston (130) lorsque la tige de piston est insérée à l'intérieur du piston pour le fonctionnement de la seringue.

17. Seringue selon la revendication 1, conçue pour être pré-remplie et stockée en étant prête à l'injection, dans laquelle :

(a) ledit fût (220) comporte une surface intérieure (221) définissant une chambre cylindrique pour maintenir un fluide injectable à l'intérieur de celle-ci ; ledit fût comportant une extrémité distale s'achevant par une pointe effilée (222) à laquelle peut être fixée une aiguille d'injection ; et une extrémité proximale (224) pour recevoir un piston (230) ;

(b) ledit piston (230) est en forme de coupelle et est monté de façon à pouvoir coulisser dans ledit fût (220), et est positionné à proximité de l'extrémité proximale (224) du fût (220) pour assurer une étanchéité avec la surface intérieure (221) du fût (220), ledit piston comprenant :

(1) une face distale convexe (232) qui sert à venir en interface avec le fluide injectable contenu dans le fût (220) ;

(2) une face proximale (234) ;

(3) une paroi extérieure (236) contiguë à la face distale convexe (232), comportant sur celle-ci : une bague distale (240), une bague proximale (241) et une bague centrale (242) s'étendant radialement vers l'extérieur et formant un joint coulissant avec la surface intérieure (221) du fût (220) ;

(4) une paroi intérieure (238) comportant des filetages femelles sur celle-ci ; et

(5) un rebord inférieur (239), qui, avec la paroi intérieure, définit une ouverture circulaire dans le piston en forme de coupelle (230), à travers laquelle un cylindre d'actionnement de piston est inséré pour son engagement ; ladite seringue comprenant de plus :

(c) un cylindre d'actionnement de piston (250) comportant une extrémité distale (252) et une extrémité proximale (260), pour s'engager avec le piston, comprenant :

(1) des filetages mâles (270) à l'extrémité distale (252) pour coopérer avec des filetages femelles dans le piston (230) ; et

(2) une poignée (262) à l'extrémité proximale ; et

(d) ladite tige de piston (280) comporte une extrémité distale et une extrémité proximale, fixées à l'intérieur dudit cylindre d'actionnement de piston, comprenant :

(1) une pointe de forme semi-circulaire (282) à l'extrémité distale, avec une face convexe faisant saillie dans la direction du piston (230), et dont le diamètre est sensiblement inférieur au diamètre du piston, de façon à appuyer contre la face proximale (234) du piston lorsqu'une pression est exercée sur la tige de piston (280) ;

(2) une molette à l'extrémité proximale (286), disposée à l'extérieur du cylindre d'actionnement de piston (250) et servant comme premiers moyens d'arrêt pour la tige de piston (280) pour limiter la saillie de la tige de piston à l'intérieur de la face proximale du piston ; et

(3) un flasque (284), également à l'extrémité proximale mais espacé de la molette (280), et disposé à l'intérieur du cylindre d'actionnement de piston (250), et servant de deuxièmes moyens d'arrêt pour la tige de piston (280), pour limiter le déplacement de la tige de piston dans la direction allant vers l'extrémité proximale du cylindre d'actionnement de piston ;

lesdits premiers moyens d'arrêt et lesdits deuxièmes moyens d'arrêt sont conçus pour limiter le déplacement de la tige de piston (280) à l'intérieur du cylindre d'actionnement de piston (250) à une longueur prédéterminée définie par la distance entre les premiers et deuxièmes moyens d'arrêt.

18. Seringue selon l'une quelconque des revendications précédentes, dans laquelle ledit fût de seringue (20, 120, 220) est constitué par un matériau imperméable aux gaz inertes, sélectionné parmi le groupe comprenant le verre, le polyéthylène, le polypropylène, les polystyrènes, les polymères acryliques et les polymères méthacryliques.

19. Seringue selon l'une quelconque des revendications précédentes, dans laquelle ledit piston (30, 130, 230) est réalisé en un matériau élastomère compressible.

FIG.1.

*FIG.2.*

*FIG.3.*

EP 0 758 255 B1

FIG.4.

FIG.5.

FIG.6.

23

FIG. 7

FIG. 8

EP 0 758 255 B1

*FIG. 9*

*FIG. 10*

FIG. 11

FIG. 12

FIG. 13

FIG. 14

FIG. 15

## FIG.16

162

164 166 152 150

## FIG.17

168

## FIG.18

172
168
174

## FIG.20

130'

134'

164'

140' 142' 141'

139'

150'

FIG.19

121'  120'  139'  130'  164'  132'  134'

EP 0 758 255 B1

FIG. 21

FIG. 22

*FIG.23*

270

250

262

252

260

*FIG.24*

282

280

284

286

*FIG.25*

250

270

239

280

FIG. 26

*FIG.27*

*FIG.28*

FIG.29